# EUROPEAN PATENT SPECIFICATION

(11) **EP 2 961 323 B1**
(45) Date of publication and mention of the grant of the patent: **09.11.2022**
(21) Application number: 14756632.7
(22) Date of filing: 26.02.2014
(51) Int. Cl.: A61B 8/08, A61B 17/34

(54) **COUPLING STRUCTURES FOR AN ULTRASOUND PROBE**
KOPPLUNGSSTRUKTUREN FÜR EINE ULTRASCHALLSONDE
STRUCTURES DE COUPLAGE POUR SONDE ULTRASONORE

(30) Priority: 26.02.2013 US 201361769676 P
(43) Date of publication of application: 06.01.2016
(73) Proprietor: C. R. Bard, Inc., Franklin Lakes, NJ 07417 (US)
(72) Inventor: LINDEKUGEL, Eric, W., Salt Lake City, UT 84106 (US); FARNWORTH, Charles, L., Riverton, UT 84065 (US); LOEWEN, John, L., Salt Lake City, UT 84124 (US)
(74) Representative: Hoffmann Eitle
(86) International application number: PCT/US2014/018681
(87) International publication number: WO 2014/134171

(56) References cited:
- WO-A2-2008/024515
- JP-A- H0 213 439
- JP-A- 2004 313 271
- US-A- 4 867 169
- US-A- 5 490 522
- US-A- 5 522 878
- US-A- 5 626 554
- US-A1- 2010 179 429
- US-A1- 2011 313 293
- US-A1- 2011 313 293
- US-B1- 6 361 499

## Description

### Technical field

The present invention relates to a cover for an ultrasound probe.

### Background art

US 2011/0313293 A1 discloses, for example, an assembly such as that shown in Figs. 24A-C.

### BRIEF SUMMARY

The invention is defined by claim 1. Preferred embodiments are defined in the dependent claims.

Briefly summarized, embodiments of the present disclosure are directed to a probe cap for use with an ultrasound probe including a head portion and an acoustic surface. In one embodiment, the probe cap includes a body that defines a cavity sized for releasably receiving the head portion of the probe therein. The probe cap body further defines a hole that is proximate the acoustic surface of the head portion. A compliant spacer component is disposed in the hole. The spacer component can include a hydrogel and provides an acoustic path between the acoustic surface and a tissue surface of a patient. The spacer component further includes a skin contact surface that defines a concavity and is deformable against the skin. The skin contact surface can further define one or more spacer elements adjacent the concavity for distributing the load of the probe pressing against the skin and preventing compression of subcutaneous structures of the patient.

In another embodiment, an ultrasound imaging system for imaging a subcutaneous structure of a patient is disclosed and includes a display, an ultrasound probe including an acoustic surface from which ultrasound signals are emitted, and first and second spacer elements. The spacer elements are positioned proximate opposite ends of the acoustic surface and are configured to provide a gap between the acoustic surface and a tissue surface of the patient. So configured, the spacer elements prevent compression of the subcutaneous structure of the patient.

In addition, embodiments to be further described below disclose various probe cap and accompanying needle guide designs for use in assisting a clinician with ultrasound probe use and needle insertion into a patient. In yet other embodiments, various coupling structures are described for providing an ultrasonic coupling between the ultrasound probe head and the patient's skin.

These and other features of embodiments of the present invention will become more fully apparent from the following description and appended claims, or may be learned by the practice of embodiments of the invention as set forth hereinafter.

### BRIEF DESCRIPTION OF THE DRAWINGS

A more particular description of the present disclosure will be rendered by reference to specific embodiments thereof that are illustrated in the appended drawings. It is appreciated that these drawings depict only typical embodiments of the invention and are therefore not to be considered limiting of its scope. Example embodiments of the invention will be described and explained with additional specificity and detail through the use of the accompanying drawings in which:
FIGS. 1A and 1B are perspective and side views, respectively, of an ultrasound probe including spacer elements;
FIG. 2 is a simplified cross sectional view of the ultrasound probe of FIGS. 1A and 1B used to image a vessel of a patient;
FIG. 3 is a side view of the ultrasound probe of FIGS. 1A and 1B enclosed within a sheath;
FIGS. 4A and 4B are side views of a portion of an ultrasound probe including spacer elements and further showing examples of possible acoustic surface configurations;
FIG. 5 is a side view of a portion of an ultrasound probe including a spacer element;
FIG. 6 shows ultrasound spacer elements;
FIG. 7 shows ultrasound spacer elements;
FIG. 8 shows ultrasound spacer elements;
FIGS. 9A and 9B show spacer elements;
FIG. 10 is a side view of an ultrasound probe including spacer elements;
FIG. 11 is a side view of an ultrasound probe including a cap including spacer elements and a sheath;
FIG. 12 is a perspective view of a spacer component;
FIGS. 13A-13C show use of the spacer component of FIG. 12;
FIG. 14 is a side view of a spacer component;
FIGS. 15A-15B show use of the spacer component of FIG. 14;
FIG. 16 is an exploded perspective view of an ultrasound probe and a probe cap;
FIGS. 17A-17D are various views of the probe cap of FIG. 16;
FIGS. 18A and 18B are an exploded perspective view and cross sectional side view of an ultrasound probe/probe cap and a spacer component, respectively;
FIG. 19 is a cross sectional view of a head portion of the ultrasound probe of FIG. 16;
FIG.20 is a cross sectional view of the probe cap of FIG. 16;
FIG.21 is a cross sectional view of a head portion of the ultrasound probe of FIG. 16 received within the probe cap of FIG. 16;
FIG. 22 is another cross sectional view showing a head portion of the ultrasound probe of FIG. 16 received within the probe cap of FIG. 16;
FIG. 23 is a perspective view of a mated configuration of the ultrasound probe and probe cap of FIG. 16;
FIGS. 24A and 24B are front and side views, respectively, of an ultrasound probe and accompanying probe cap including a compliant spacer component;
FIG. 24C is a perspective view of the probe cap of FIGS. 24A and 24B;
FIGS. 25A-25D are various views of a probe cap;
FIGS. 26A and 26B are various exploded views of a probe cap;
FIG. 27 is a side view of the probe cap of FIGS. 26A and 26B shown in contact with a patient's skin above a subcutaneous vessel;
FIGS. 28A and 28B are perspective and cross sectional views, respectively, of a probe cap;
FIGS. 29A-29D are various views of a probe cap assembly;
FIGS. 30A and 30B are various perspective views of a probe cap;
FIG. 31 is a cross sectional side view of the probe cap of FIGS. 30A and 30B shown attached to an ultrasound probe;
FIG. 32 is a perspective view of a probe cap;
FIGS. 33A and 33B are partial cross sectional side views of an ultrasound probe and probe cap;
FIG. 34 is a perspective view of a needle guide according to one embodiment; and
FIGS. 35A and 35B are side and perspective views, respectively, of the needle guide of FIG. 34 attached to a probe cap.
FIG. 36 is a side view of a coupling structure for an ultrasound probe according to the present invention;
FIGS. 37A and 37B show various views of coupling components for an ultrasound probe;
FIG. 38 is a cross sectional view of a probe cap including one of the coupling components from the structures shown in FIGS. 37A and 37B;
FIG. 39 is a perspective view of a reinforcement structure for use with a coupling component;
FIG. 40 is a cross sectional view of a probe cap including a coupling component and a reinforcement structure;
FIG. 41 is a top view of a coupling structure for an ultrasound probe;
FIG. 42 is a perspective view of a probe cap including a coupling component;
FIGS. 43A and 43B are various views of a coupling component;
FIG. 44 is a cross sectional view of a probe cap;
FIG. 45 is a top view of an array of probe caps such as those shown in FIG. 44;
FIGS. 46A and 46B are various views of a probe cap; and
FIG. 47 is a cross sectional view of a probe cap.

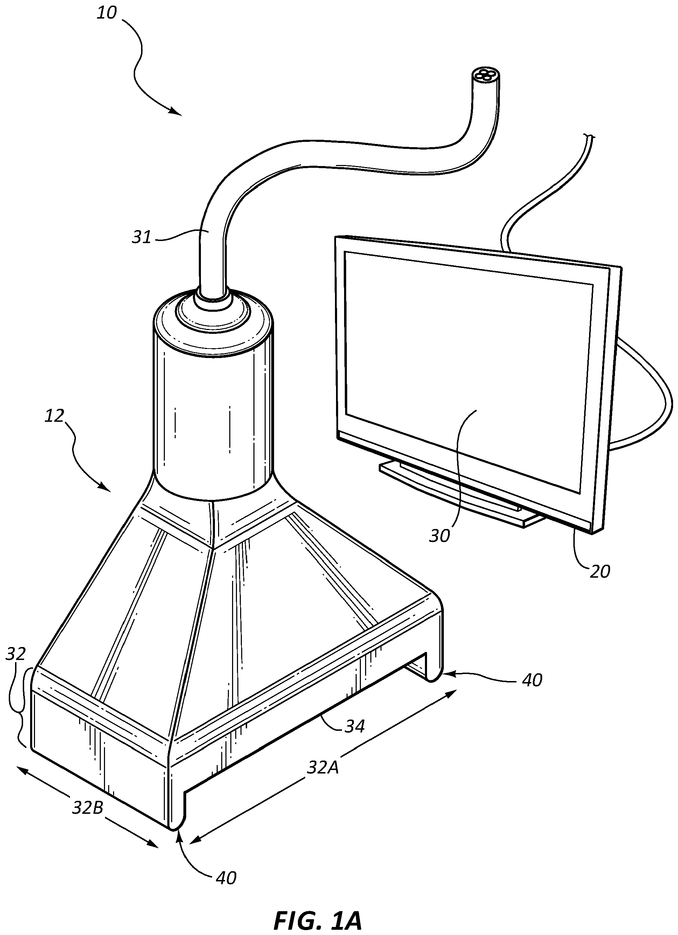

The embodiments illustrated in figures 1A-35B and 37A-47 are not according to the invention and are present for illustration purposes only.

### DETAILED DESCRIPTION OF SELECTED EMBODIMENTS

Reference will now be made to figures wherein like structures will be provided with like reference designations. It is understood that the drawings are diagrammatic and schematic representations of exemplary embodiments of the present invention, and are neither limiting nor necessarily drawn to scale.

For clarity it is to be understood that the word "proximal" refers to a direction relatively closer to a clinician using the device to be described herein, while the word "distal" refers to a direction relatively further from the clinician. For example, the end of a catheter placed within the body of a patient is considered a distal end of the catheter, while the catheter end remaining outside the body is a proximal end of the catheter. Also, the words "including," "has," and "having," as used herein, including the claims, shall have the same meaning as the word "comprising."

Embodiments described below are generally directed to various components for spacing an acoustic surface of an ultrasound probe from a tissue surface of a patient during ultrasound procedures to image subcutaneous tissues of the patient. Such ultrasound procedures are employed, for instance, in connection with the placement of a catheter within a vessel of the patient. As will be described, the components for spacing the acoustic surface in one embodiment prevent undesired compression of subcutaneous vessels, especially superficial vessels, which in turn improves the imaging of such vessels by the probe. In addition, embodiments to be described further below disclose various probe cap and accompanying needle guide designs for use in assisting a clinician with ultrasound probe use and needle insertion into a patient.

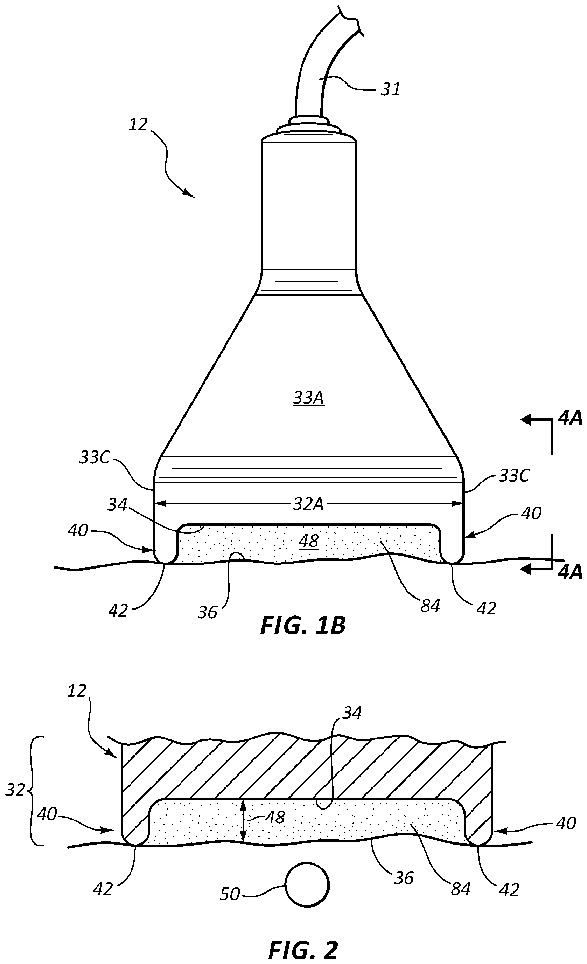

Reference is first made to FIGS. 1A and 1B, which depict an ultrasound imaging system 10, including an ultrasound probe 12 and a console 20 including a display 30 for depicting an image produced by the probe. In the present embodiment, the probe 12 is operably connected to the console 20 via a cable 31, though in one embodiment the probe can be wirelessly connected thereto.

The probe 12 includes a head 32 defined by a longitudinal length 32A and a width 32B. The body of the probe generally defines a front face 33A, a rear face 33B, and side faces 33C. It should be appreciated that the preceding description of the probe is not meant to limit application of the principles described herein in any way. The probe head 32 includes an acoustic surface 34 extending along at least a portion of a longitudinal length 32A of the probe head from which ultrasonic impulses are emitted in order to penetrate and image subcutaneous portions of the patient. Note that the size, shape and configuration of both the probe and acoustic surface can vary from what is described herein while still residing within the principles of the present disclosure. Note also that FIG. 1A shows just one example of an ultrasound imaging system; other systems including other components can also benefit from the principles described herein.

As depicted in FIGS. 1A and 1B, the probe head 32 includes two spacer elements, generally depicted at 40, disposed adjacent the probe acoustic surface 34 at each end of the longitudinal length 32A. Each spacer element 40 acts as an extended surface to provide a gap 48 between the acoustic surface 34 and the skin 36 or other tissue surface of the patient, as further described below, when the probe 12 is placed on the patient's skin for use in subcutaneous imaging.

In greater detail, each spacer element 40 defines a blade-like extended surface that includes a contact surface 42 for contacting the tissue/skin 36 of the patient. The contact surface 42 can be shaped in one of several configurations, as will be discussed further below.

Reference is now made to FIG. 2. When no spacers are present on an ultrasound probe, the acoustic surface thereof directly contacts the patient's skin during imaging, which can cause a downward pressure sufficient to undesirably compress a subcutaneous vessel disposed beneath the probe. Further, the proximity of the probe acoustic surface to the patient's skin can cause the focal point of the probe to reside below the vessel to be imaged, resulting in less than optimal image resolution of superficial vessels or other objects residing relatively close to the skin surface.

In contrast to the above, FIG. 2 shows the probe 12 including the spacer elements 40 disposed at each longitudinal end of the probe head 32 and adjacent the acoustic surface 34. So configured, the acoustic surface 34 is spaced apart from the patient's skin 36 during probe use, and only the contact surfaces 42 of the spacer elements 40 are in contact therewith. The gap 48 is thus defined between the acoustic surface 34 and the patient's skin 36, which can be filled with an ultrasonic gel 84 or other acoustically transparent substance to improve imaging, in one embodiment.

Because the acoustic surface 34 of the ultrasound probe head 32 is not in direct contact with the patient's skin 36 during probe use, pressure on the skin imposed by the acoustic surface is avoided, which in turn prevents a vessel 50 underneath the probe 12 from being compressed by the probe during use. Instead, any downward force provided by the probe 12 is directed through the spacer elements 40. As such, the vessel 50 below the acoustic surface 34 remains patent and can be accurately imaged. Further, the increased distance between the acoustic surface 34 and the patient's skin 36 provided by the gap 48 moves the focal spot of the probe 12 to a location relatively close below the skin surface, which enables superficial vessels and other objects residing near the skin surface to be brought more closely to the focal point of the probe and be sharply imaged.

Note that the gap 48 shown in FIGS. 1A-2 is bounded during probe use by the acoustic surface 34, the skin 36, and the spacer elements 40. As such, the gap 48 remains open below the front and rear faces 33A, 33B of the probe 12. Note that additional spacers could be employed to further define the gap 48, if desired.

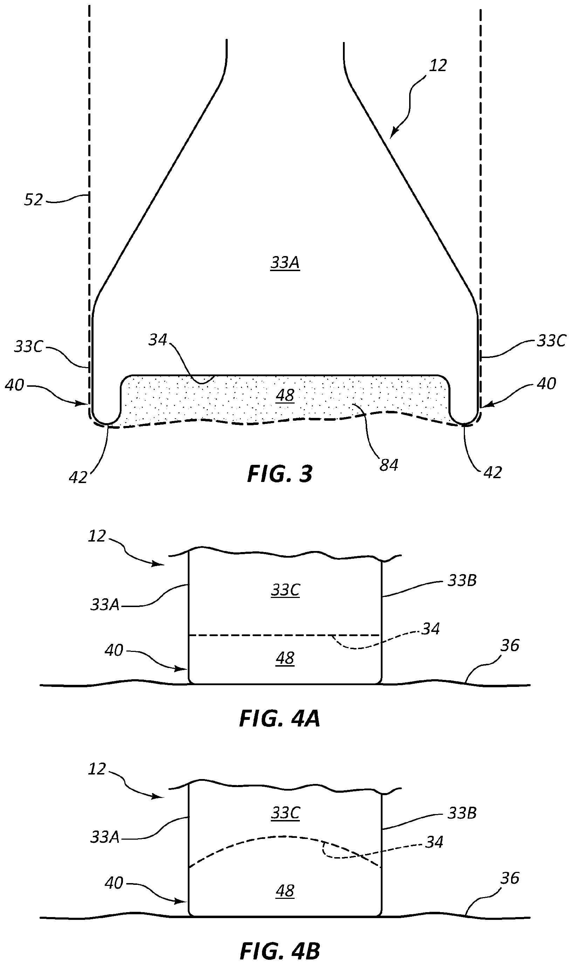

Reference is now made to FIG. 3, wherein a sheath 52 is placed over the probe 12 to provide a sterile field about the probe. The sheath 52 can be disposed about the probe 12 such that a relatively close fit is defined between the sheath and the side faces 33C and front/rear faces 33A, 33B of the probe so that the ultrasound gel 84 can be included in and confined within the gap 48 by the sheath and the spacer elements 40. Note that sheaths or barriers of many different styles or configurations may be used.

FIGS. 4A and 4B show example surface configurations for the acoustic surface 34. In FIG. 4A, the acoustic surface 34 is flat as to be substantially parallel with the patient's skin 36 during probe use. In FIG. 4B, the acoustic surface 34 defines a concave shape with respect to the skin 36. This configuration can assist in trapping a volume of ultrasound gel within the gap 48. Of course, other acoustic surface configurations can be employed.

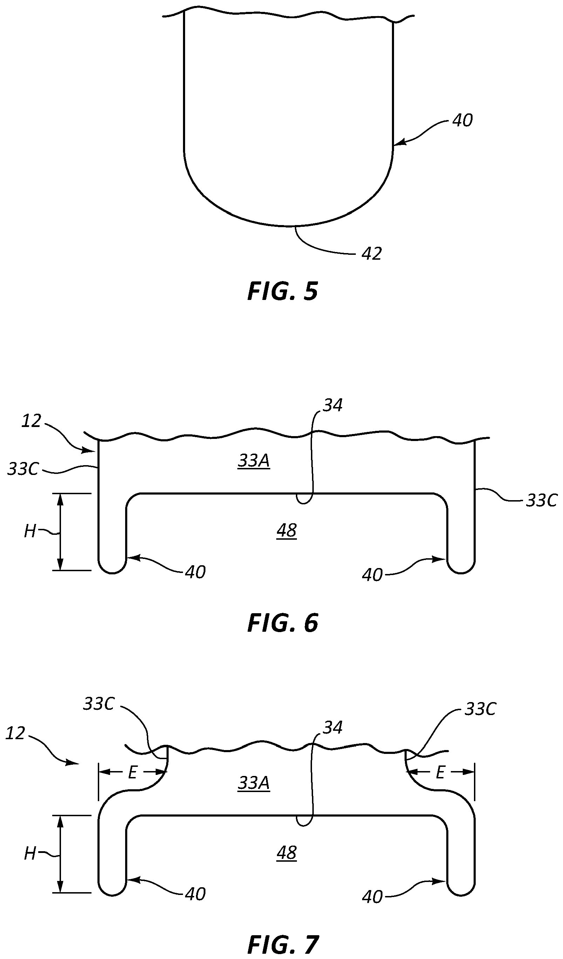

FIG. 5 gives one example of a possible configuration for the contact surface 42 of the spacer element 40, wherein the contact surface defines a convex shape for engagement with the patient's skin or other tissue surface. Note this is in contrast to the relatively flat contact surface 42 shown in FIGS. 4A and 4B, for instance. Other spacer contact surface shapes can be employed, including straight, rounded, angled, etc.

FIG. 6 shows that a height "H" of each spacer element 40 can be defined according to a particular need or application in order to define a particular separation between the acoustic surface 34 and the patient's skin 36 during use of the probe 12. Note that in one embodiment, the spacer elements are integrally formed with the probe housing. In another example, the spacer elements are removably attached to the probe. The spacer elements can include materials similar to or different from those materials included in the probe housing.

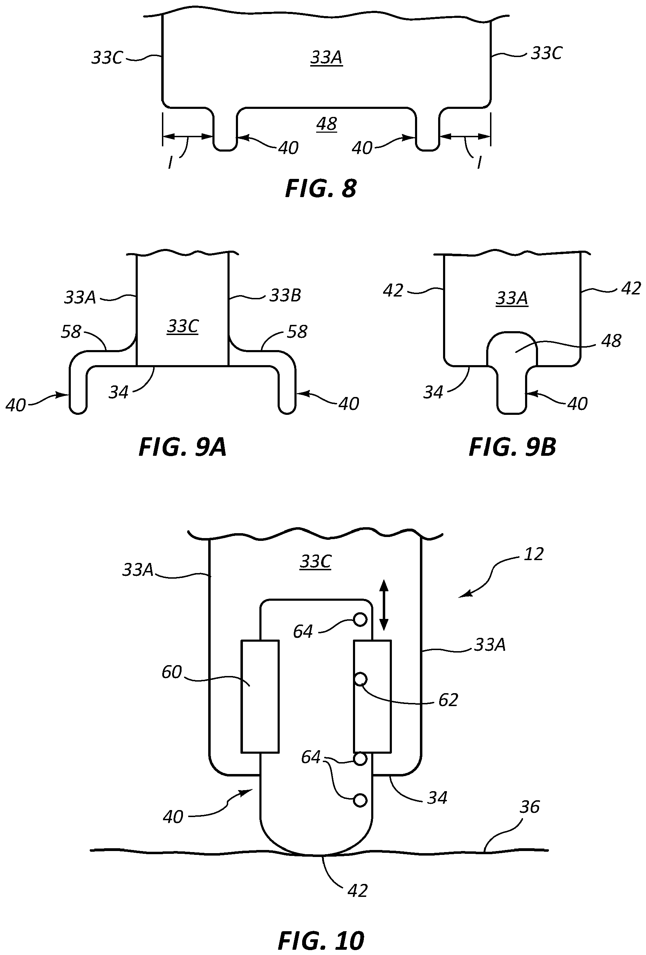

Reference is now made to FIGS. 7 and 8, wherein FIG. 7 shows that in one embodiment the spacer elements 40 can be configured to extend longitudinally a distance "E" past the side surfaces 33C of the probe 12. In FIG. 8, each of the spacer elements 40 is inset a distance "I" from the probe side surfaces 33C.

FIGS. 9A and 9B depict yet another possible spacer element configuration, wherein each spacer element 40 is included at an end of an extension arm 48 that extends from a corresponding one of the front and rear faces 33A, 33B of the probe 12. Such a configuration may be useful, for instance, in advancing the probe 12 along the patient skin 36 in a direction parallel to the longitudinal length of the acoustic surface 34. These and other spacer configurations are therefore contemplated as residing with the spirit of the present disclosure.

FIG. 10 shows a height-adjustable spacer element 40 so as to allow variation in the set-off distance of the acoustic surface 34 from the skin 36. In the illustrated embodiment, a bracket 60 that slidably receives the spacer element 40 is included on the side face 33C of the probe 12 and includes a depression or hole 62. Corresponding protuberances 64 are included on the spacer element 40 and are configured to be selectively received into the hole 62 so as to removably lock the spacer element in place at a specified height. The protuberances 64 are distributed along the length of the spacer element 40 such that one of multiple spacer heights may be selected. A similarly adjustable spacer element is included on the opposite side face of the probe 12. Of course, other adjustable spacer element configurations can be included on the probe in addition to that explicitly described here.

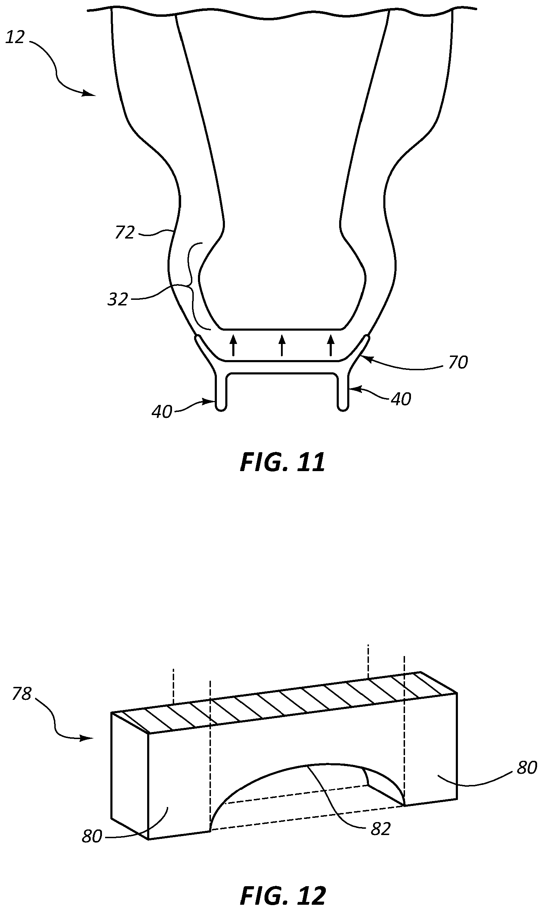

FIG. 11 shows details, wherein the spacer elements 40 are included on a cap 70 that is removably attachable to the probe head 32. In the present embodiment, the cap is snapped on to the probe head 32 via an interference fit, but in other embodiments other attachment schemes can be employed, including inter-engaging surfaces on the probe and cap, for example. A sheath 72 is attached to the cap 70 so as to provide a sterile barrier for the ultrasound probe 10. The cap 70 and sheath 72 may be disposable.

It should be appreciated that the number, size, height, shape, etc., of the spacer elements can vary from what is explicitly described herein. For instance, one, three, or more spacers can be included. Or the relative heights of the spacers can differ one from another so as to produce an angled probe-to-skin configuration. The probe can include one of many different shapes, designs, etc. These and other modifications are thus considered part of the present disclosure.

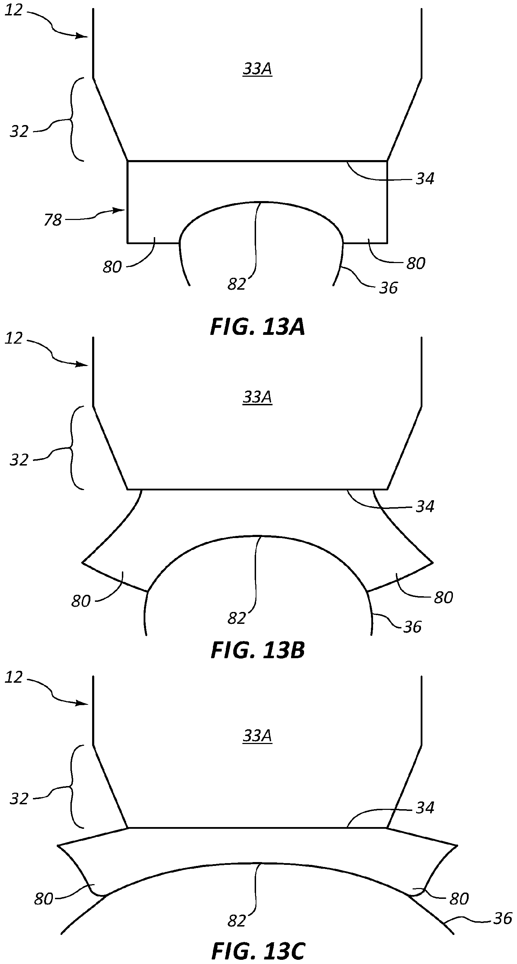

FIG. 12 depicts details of a spacer component 78 configured for attachment to the probe head 32, as shown in FIG. 13A. The spacer component 78 includes a body of compliant material, such as a hydrogel, in one embodiment, which generally maintains its intended shape when deforming forces are absent. The compliant material in one embodiment can include AQUAFLEX^{®} ultrasound gel from Parker Laboratories, Inc., Fairfield, New Jersey. The spacer component 78 further defines spacer elements 80 on each longitudinal end thereof, with a concavity 82 defined between the spacer elements. It is appreciated that other suitable materials can be employed for the compliant material of the spacer component, including acoustically transparent, sufficiently solid materials such as soft silicone, rubber, etc. The compliant material may be thermoformable, sterilizable, and shelf stable for at least one year.

As shown in FIGS. 13A-13C, the spacer component 78 due to its compliant nature can deform so as to conform to the shape of the surface of the patient's skin 36 during use of the probe 12. For example, the probe 12 including the spacer component 78 can be placed on a patient's arm. So positioned, the spacers 80 of the spacer component 78 can deform as needed as to match the cross sectional curvature of the arm surface and maintain contact with the skin 36 thereof. FIGS. 13B and 13C show such deformation of the spacer component 78 for relatively larger arms. Thus, the spacer component 78 provides an acoustic path between the acoustic surface and the skin surface without need of a flowable ultrasound gel. It is appreciated that the spacer component can be used in connection with imaging other portions of the patient's body and that the spacer component can define other shapes for contacting differently shaped body portions. Further, an ultrasound gel can be included between the spacer component and the skin, such as in the concavity thereof.

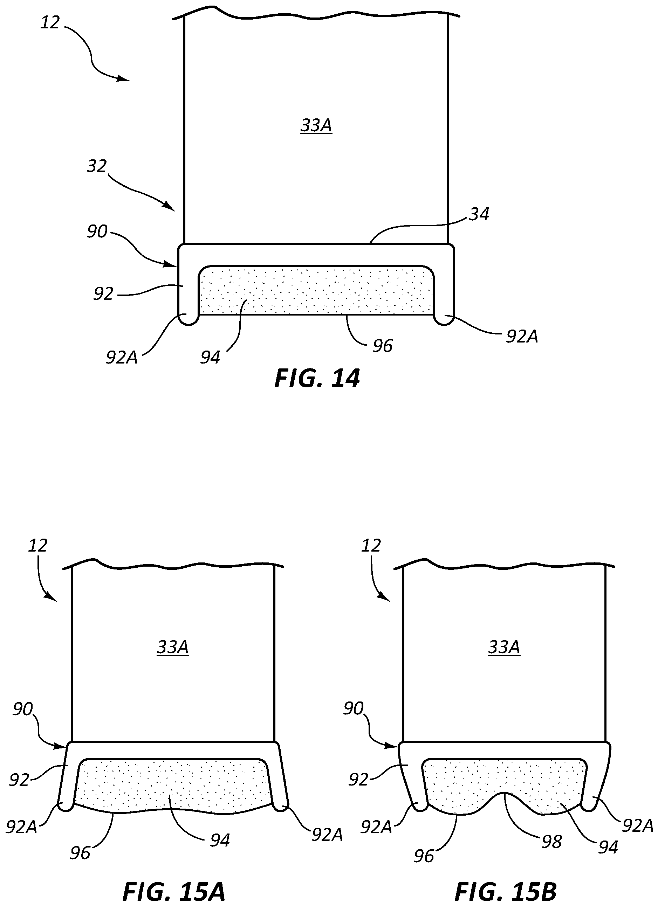

FIG. 14 depicts a spacer component 90, including a flexible casing 92 that can operably attach to the probe head 32, as shown. The casing 92 includes arms 92A that contain a compliant insert 94, such as hydrogel in one embodiment. As shown in FIGS. 15A and 15B, the spacer component 90 is positioned on the probe head 32 so as to provide both spacing and an acoustic path between the acoustic surface 34 and the surface of the skin 36 or other tissue surface such that flowable ultrasound gel is not needed. So configured, the insert 94 thereof defines a contact surface 96 for contacting the surface of the skin 36 during ultrasound probe use. In one example, the arms 92A of the casing 92 can be pressed inward to modify the shape of the contact surface 96. For instance, FIG. 15A shows that the contact surface 96 of the insert 94 defines a relatively shallow concavity 98 when the arms 92A of the casing 92 are allowed to flex outward. When the arms 92A are pressed inward as in FIG. 15B, however, the insert 94 is compressed by the arms and the concavity 98 of the contact surface 96 becomes relatively more pronounced. Such a configuration of the contact surface 96 may be desirable to stabilize a position of the subcutaneous vessel while preventing its collapse. The arms 92A can be biased to restore themselves to a given position when not being pressed by a user.

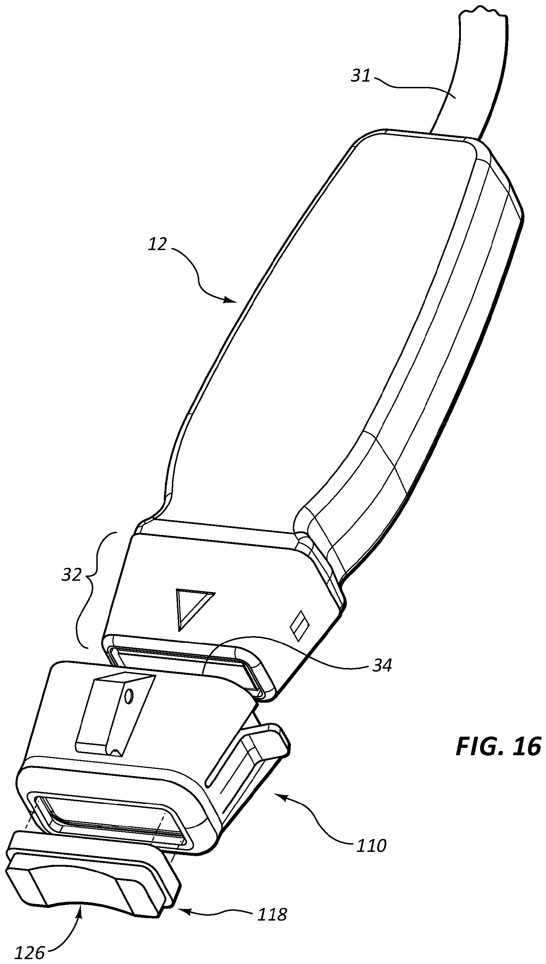

FIG. 16 shows details of a probe cap 110 for use with the probe 12. The cap 110 is configured to receive therein the head 32 of the probe 12 and to provide a spacer component 118 for providing desired spacing between the acoustic surface 34 of the probe head 32 and the skin 36.

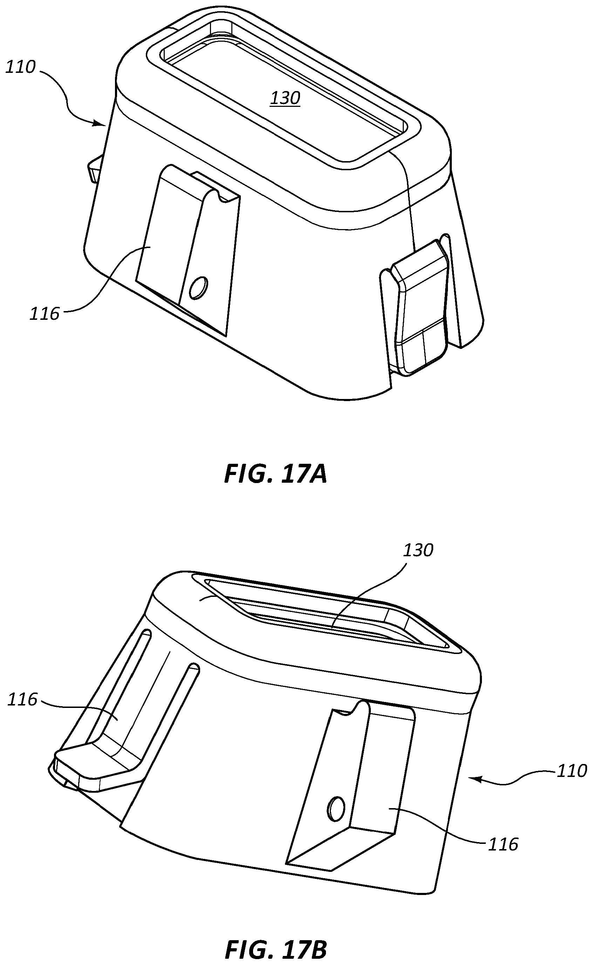

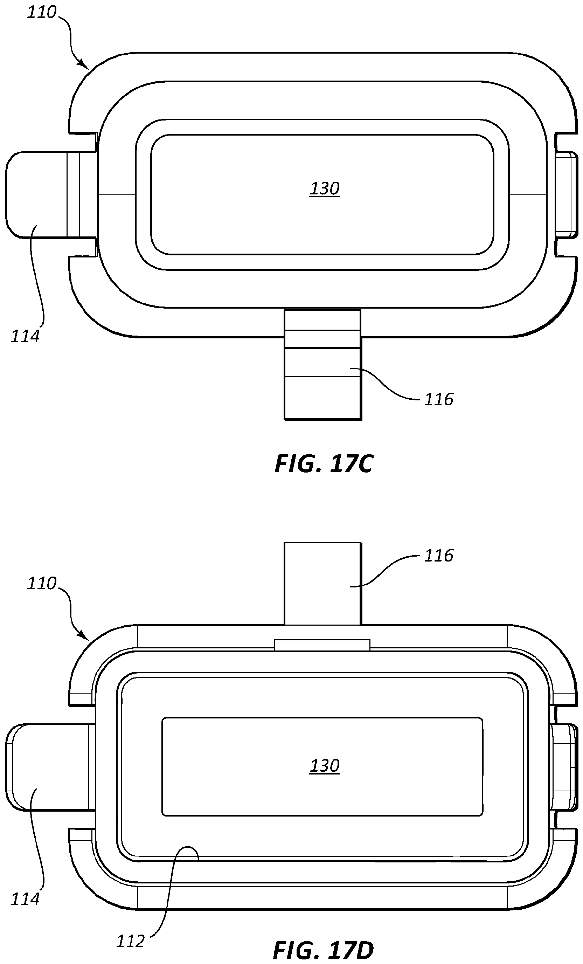

As shown in FIGS. 17A-17D, the cap 110 defines a cavity 112 that is sized to receive therein the head 32 of the probe 12. An engagement feature 114 is included with the cap 110 to releasably and mechanically attach the cap to the probe 12, though it is appreciated that various designs can be employed to accomplish the same functionality. The cap 110 further includes a needle guide base 116 on which a detachable needle guide can be placed so as to assist a clinician in placing a needle through the skin 36 after a vessel has been located through use of the ultrasound system 10 (FIG. 1A).

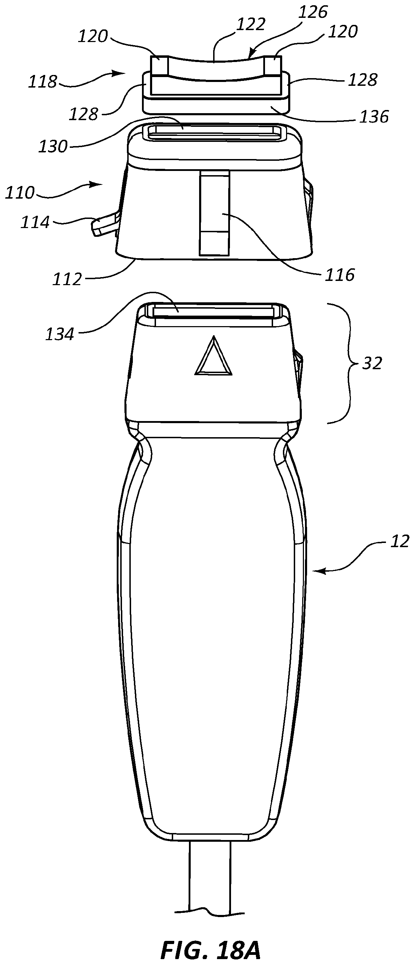

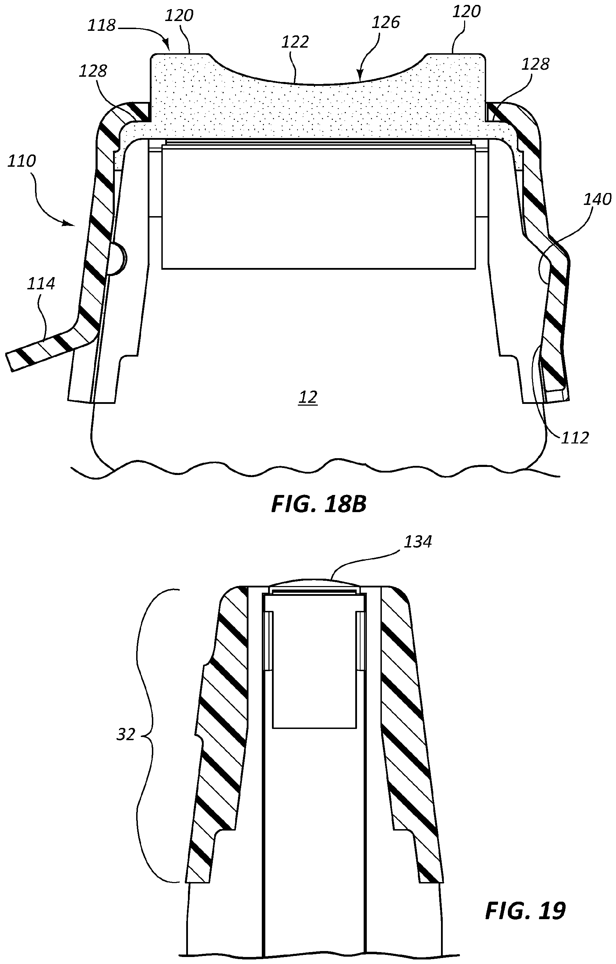

With continuing reference to FIGS. 17A-17D, reference is made to FIGS. 18A and 18B, which depict various details of the spacer component 118, which is disposed in a hole 130 defined in the cap 110, best seen in FIGS. 17A and 17C. As shown, the spacer component 118 includes a skin contact surface 126 that defines two spacer elements 120 and a concavity 122 disposed therebetween. The spacer component includes 118 a compliant material, such as hydrogel in one embodiment, though it is appreciated that other suitable materials can also be employed. The spacer component 118 thus requires no use of flowable ultrasound gel to be applied to the skin 36 in order to provide an acoustic path between the acoustic surface 134 and the patient's skin. The spacer component 118 further defines a lip 128 about a perimeter thereof to assist in its retention within the hole 130 of the cap 110, as seen in FIG. 18B. As shown, in the present embodiment the lip 128 is shaped so as to be sandwiched between the cap 110 and probe head 32, thus preventing its unintended removal from the cap.

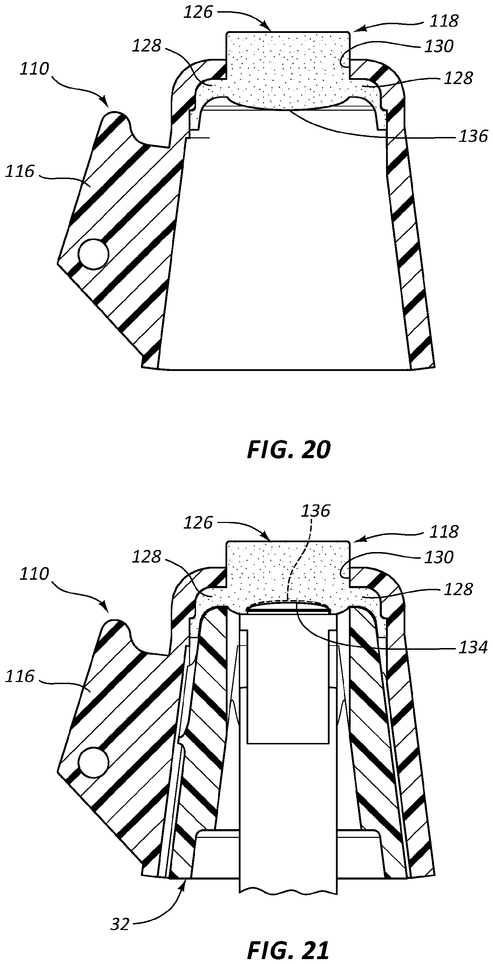

FIG. 19 shows that the acoustic surface 134 of the probe head 32 defines a convex shape. Correspondingly, FIG. 20 shows that a probe contact surface 136 of the compliant spacer component 118 also defines a convex surface. FIG. 21 shows that when the probe head 32 is received into the cavity 112 of the cap 110, the convexly shaped probe contact surface 136 of the spacer component 118 deformably engages the convexly shaped acoustic surface 134 of the probe head 32 so as to ensure adequate contact therebetween and to provide a suitable acoustic path through the spacer component. Of course, other complementary shapes can be employed on the acoustic surface and probe contact surface of the spacer component.

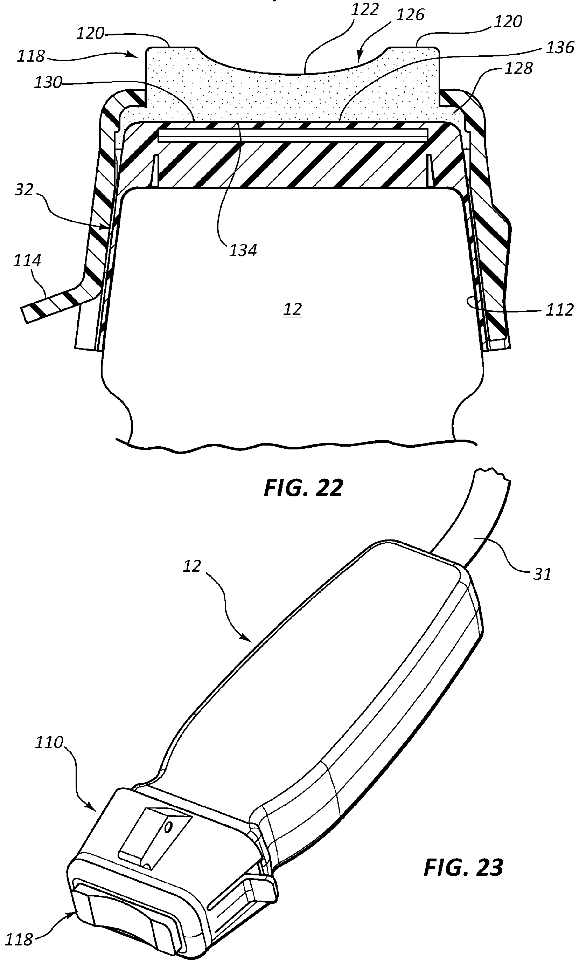

FIG. 22 shows another view of the engagement between the probe head 32 and the cap 110, according to the present example. A recess 138 is included on the cap 110 to receive therein an orientation nub 140 on the probe head 32, which nub provides a landmark for orienting an ultrasound image on the display 30 (FIG. 1A) with the orientation of the probe 12 as held by the clinician. FIG. 23 shows the cap 110, including the spacer component 118, removably attached to the probe 12. Note that in one example the cap provides a sterile barrier for the probe head, and is disposable.

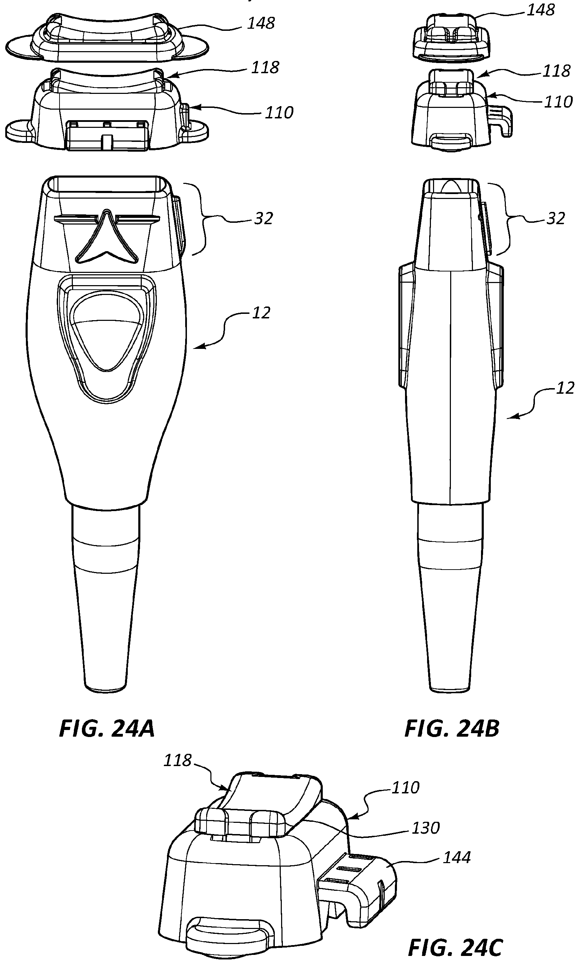

FIGS. 24A-24C depict the probe cap 110 and the concavely-shaped, compliant spacer component 118 according to one example, together with the ultrasound probe 12. As shown, a suitably shaped cover 148 is also included for covering the spacer component 118 to prevent contamination thereof and to prevent the spacer component from drying out before use. When use of the probe cap is desired the cover 148, which is fit to the probe cap 110 via a friction or other suitable fit, can be simply removed and discarded by the clinician.

As best seen in FIG. 24C, the cap 110 includes in the present example a bracket 144 to which a needle guide can be removably attached so as to enable guidance of a needle toward a desired vessel imaged by the ultrasound probe 12. Further details regarding one non-limiting example of a needle guide that can be attached to the bracket 144 can be found, for instance, in U.S. Patent Application No. 13/335,587, filed December 22, 2011, and entitled "Selectable Angle Needle Guide". Note that the needle guide and bracket can vary from what is shown and described herein.

The discussion below discusses yet other structures for enhancing use of an ultrasonic probe in connection with placement of catheters and other medical devices in the body of a patient. Indeed, the embodiments disclosed herein facilitate ease of use when ultrasonically imaging portions of the patient body in preparation for device placement therein. Examples of such placement scenarios include the insertion by a clinician of a needle, PICC catheter, PIV catheter, mid-line catheter, etc. into the patient body via a transcutaneous insertion site.

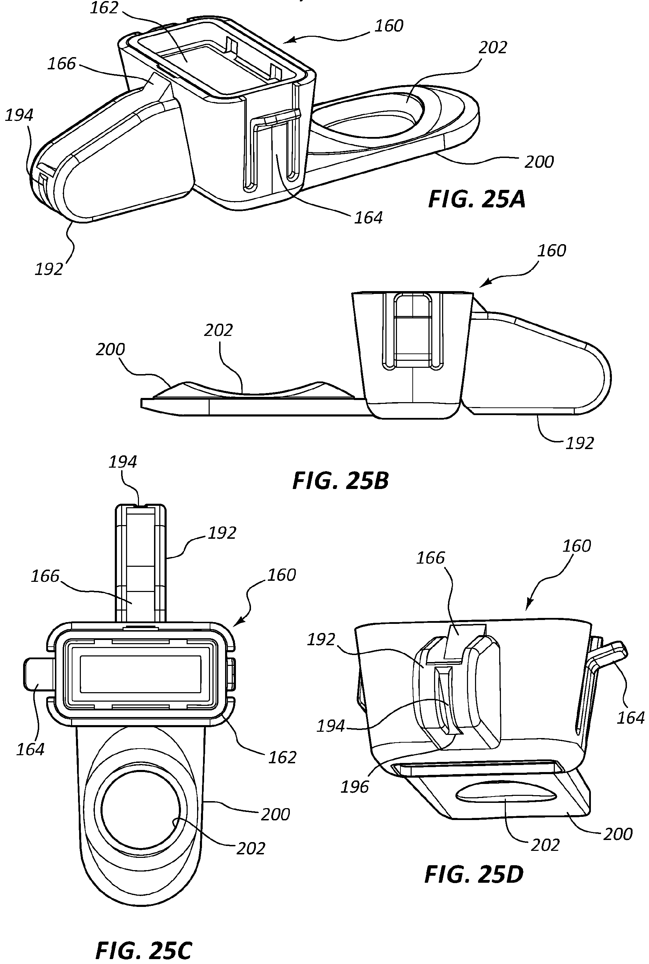

FIGS. 25A-25D show details of a probe cap 160 according to one example, which defines a cavity 162 for receiving therein the head 32 of the probe 12 and an engagement feature 164 for enabling removable engagement of the cap to the probe head. A fixture 166 is included on the side of the cap 160 and is configured for removably receiving thereon a needle guide 192. In another embodiment, this and other needle guides disclosed herein can be permanently attached to the cap. Note that, though not shown here, a spacer component similar to those shown and described in connection with FIGS. 24A-24C is disposed in the aperture 130 of the cap 160 to provide an acoustic pathway from the probe head 32 to the patient's skin.

As best seen in FIG. 25D, the needle guide 192 defines a channel 194 into which a portion of a cannula of a needle to be inserted into the patient can be temporarily received. Note that the size of the channel can accommodate needle cannulas of various sizes/diameters, as here, or can be configured to accept needles of a predetermined size. An abutment surface 196 is included at a distal end of the channel 194 about which the needle can pivot so as to continuously define differing angles of attack with respect to the patient's skin during needle insertion procedures. As such, the needle guide 192 is capable of guiding the needle at any one of a variety of angles of attack toward the patient's skin while maintaining alignment of the needle with the subcutaneous vessel being imaged by the probe 12.

Note that the probe cap 160 and other caps discussed herein can be configured to mate with the head portion of the ultrasound probe in a variety of ways, including friction fit, clip-pocket engagement, adhesively, hook-and-loop, etc. The cap portion of the probe cap can also vary in design from what is shown and described herein.

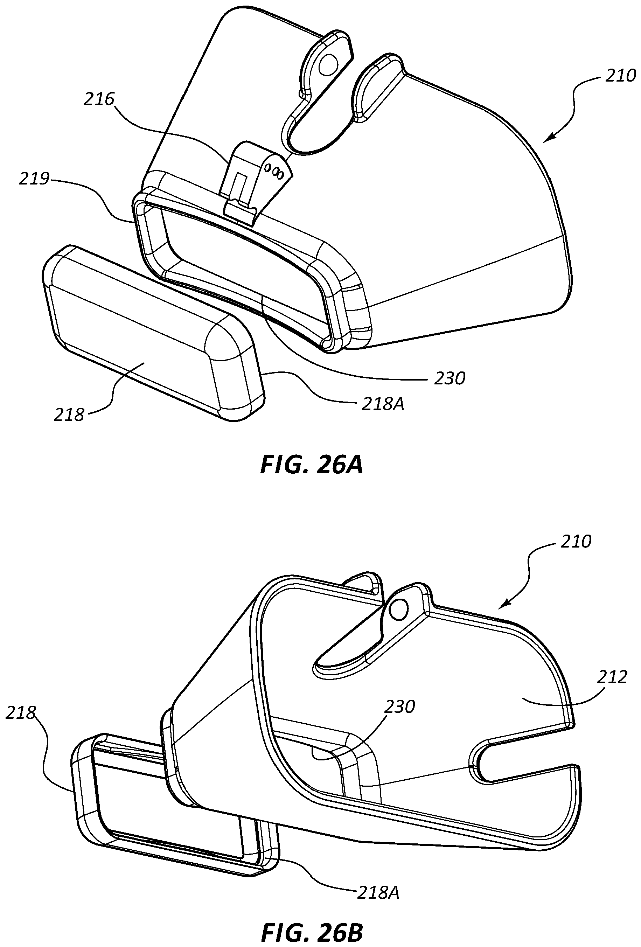

The cap 160 further includes a stabilization arm 200 extending from a distal portion of the cap body. The stabilization arm 200 is configured to rest against the skin of the patient when the cap-equipped probe is held vertically and placed against the patient's skin during ultrasound imaging procedures, thus stabilizing the probe in the vertical position. Moreover, the stabilization arm 200 can assist in securing the cap-equipped probe to the patient's skin via the use of a cord or elastic band, for instance, that is extended about the patient's arm and over the stabilization arm, thus maintaining the ultrasound probe in the upright position without manual contact by the clinician during use and providing more freedom to the clinician during the imaging procedure. A hole 202 is also defined in the stabilization arm 200 in one embodiment to enable the clinician to press the patient's skin therethrough in order to locate/occlude a subcutaneous vessel. The area proximate the perimeter of the hole 202 is contoured in the present embodiment to assist with finger placement by the clinician. FIGS. 26A and 26B show various details of a probe cap 210 according to another example, including a cavity 212 defined by the cap body that is configured to supportably receive the head 32 of the ultrasound probe 12 therein via snap-fit or other suitable modality. A fixture 216 for receiving thereon a needle guide is also included on the cap body.

A compliant membrane 218 defining a lip 218A about its perimeter is included for attachment to the cap body. Specifically, the cap body defines a ridge 219 about an aperture 230 at the distal end of the body. The lip 218A of the membrane 218 is configured to resiliently attach to the ridge 219 so as to join the membrane to the cap body and cover the ultrasound transducer of the probe head 32 when the cap 210 is attached to the probe 12. The membrane 218 thus provides an acoustic pathway between the transducer and the patient's skin. Note that ultrasound gel on the patient's skin may, but need not, be used with the cap 210 during ultrasound imaging. Note also that the membrane 218 in one embodiment includes silicone, though other suitable, compliant materials can also be employed.

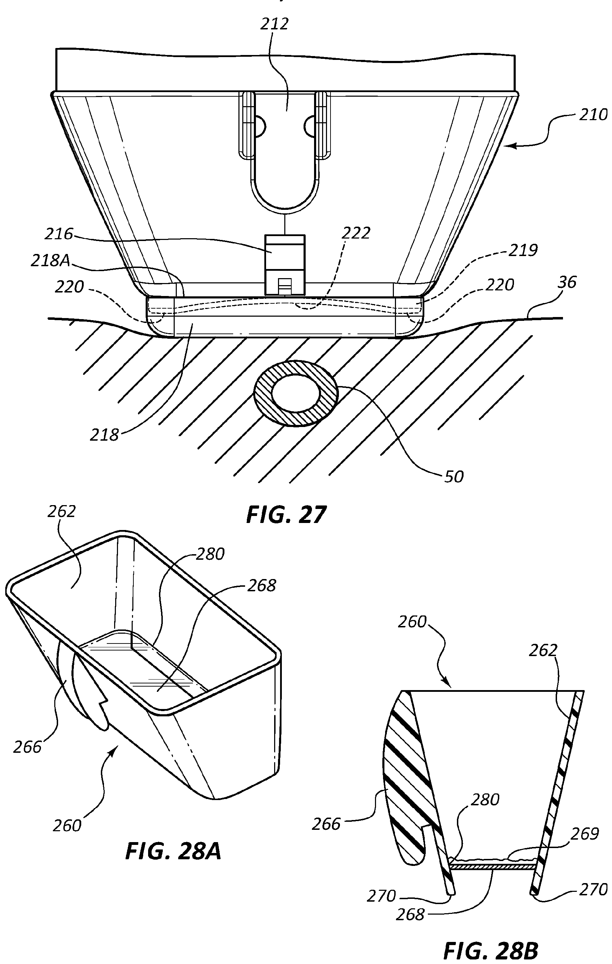

In greater detail, the ridge 219 includes a concavely shaped concavity 222, as best seen in FIGS. 26A and 27, such that it defines two standoffs, or spacers 220, on either end. The compliant nature of the membrane 218 enables it to deform to the concavity 222 of the ridge 219 when the membrane is placed against the patient's skin during ultrasound procedures. Thus, the membrane 218 can conform to the skin 36 of the patient during ultrasound imaging so as to enable imaging of subcutaneous structures, such as a superficial vessel 50 seen in FIG. 27, without providing undesired compressive forces thereon.

FIGS. 28A and 28B depict a probe cap 260 according to one example, wherein the body of the cap defines a cavity 262 and a fixture 266 for receiving thereon a needle guide. An ultrasonically transparent membrane 268 is included proximate an aperture 280 at a distal end of the cap body to cover the transducer of the head of an ultrasound probe inserted therein. An ultrasonically transmissive medium, such as ultrasound gel 269, can be placed on an interior surface of the membrane 268 to ensure acoustic coupling between the transducer and the skin of the patient. As with other cap examples described herein the probe cap 260 can be configured as a sterile cap to provide sterility or isolation for the ultrasound probe. Spacers 270 can also be included on either side of the membrane 268 to prevent compression by the cap 260 of superficial vessels when the cap 260 is placed against the skin. Note that ultrasound gel can also be placed between the membrane 268 and the patient's skin to improve signal transfer, if desired.

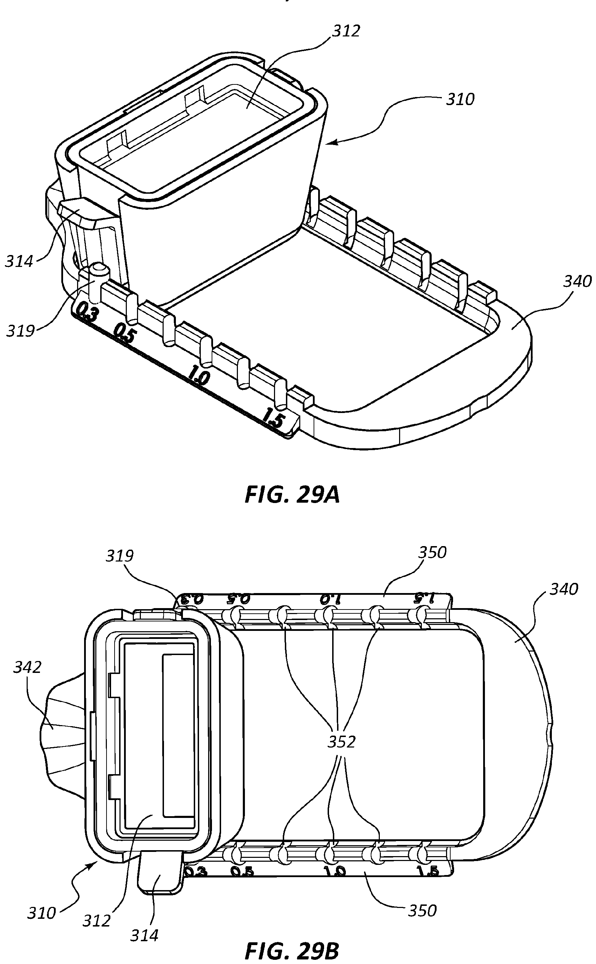

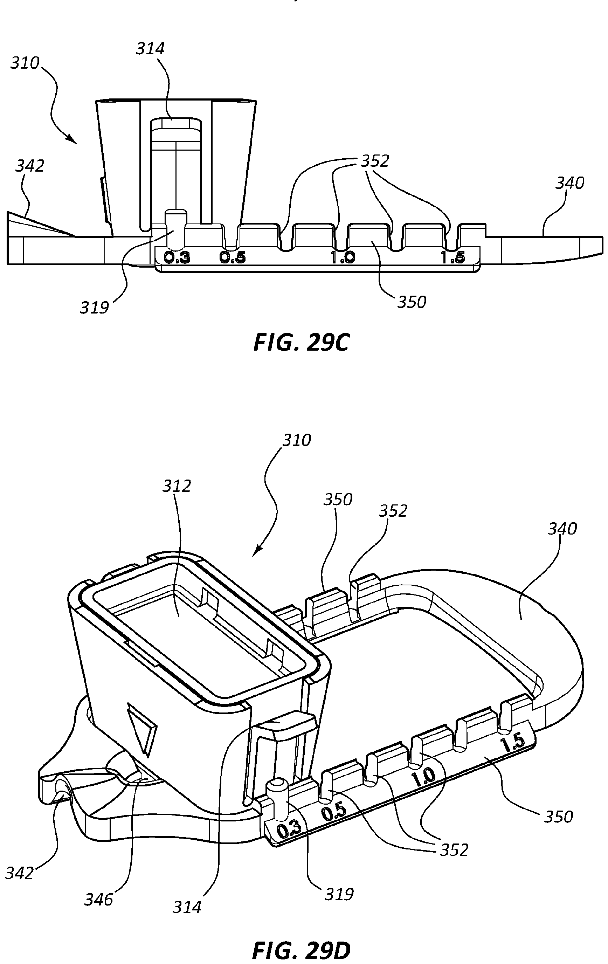

FIGS. 29A-29D depict details of a probe cap assembly 310 according to another example, wherein the assembly includes a cap body defining a cavity 312 for receiving therein the head 32 of the ultrasound probe 12, as before. Also as before, an engagement feature 314 is included to secure the cap body to the probe 12.

The cap body is movable between two parallel rails 350 of a bracket 340. Each rail 350 includes a plurality of slots 352 that align with corresponding slots on the opposing rail 350. Tabs 319 included on either longitudinal end of the cap body are configured to be selectively received into corresponding opposed slots 352 of the rails 350, as shown in FIGS. 29A-29D. In the illustrated example, the cap body is selectively repositionable along the bracket rails 350 via manual movement by lifting the cap body so as to remove the tabs 319 from the corresponding slots 352, repositioning the cap body as desired with respect to the bracket rail slots, then inserting the tabs into the selected slots. In other embodiments, it is appreciated that other modalities for moving the cap body relative to the bracket are possible, including sliding movement, gear-driven movement, etc.

As best seen in FIGS. 29B-29D, a needle guide 342 is included in the bracket to guide a needle into the patient's body when the probe cap assembly 310 is placed on the patient's skin. An observation hole 346 is also included on the bracket 340 so as to enable a clinician inserting the needle to observe blood flashback upon the needle accessing the subcutaneous vessel.

Note that the needle guide 342 in the present example is disposed at a fixed angle with respect to the bracket 340 and that the cap body is movable along the bracket with respect to the needle guide. This arrangement thus enables subcutaneous tissue to be imaged, by the ultrasound probe disposed in the cap body, at differing discrete distances from the needle guide 342. Further, this arrangement enables a needle inserted through the needle guide 342 to access an ultrasonically imaged vessel or other target at any one of a plurality of depths below the skin without the need for adjusting the angle of attack of the needle.

In greater detail, the probe 12 while disposed in the cap body of the probe cap assembly 310 can ultrasonically image a subcutaneous vessel within the patient and determine the depth below the skin surface at which the vessel resides. One or more of the slots 352 are marked with a number, indicating the depth below the skin at which a needle inserted into the patient through the needle guide 342 will intercept the subcutaneous vessel. Thus, the bracket 340 can be adjusted until the tabs 319 thereof are disposed in the slots 352 on either rail 350 corresponding to the depth of the imaged vessel. When the needle is inserted into the patient's skin through the needle guide 342, it can be advanced until it intercepts and accesses the imaged vessel at the determined depth, as desired. As such, it is appreciated that the probe cap assembly 310 can assist with needle access of an ultrasonically imaged vessel through a fixed-angle needle guide regardless of the depth of the vessel, thus obviating the need for an adjustable angle needle guide in the present embodiment. Note that the depth measurements of the bracket can vary from what is shown, but in one embodiment, the depths accessible via the probe cap assembly 310 vary from about 0.3 cm to about 1.5 cm.

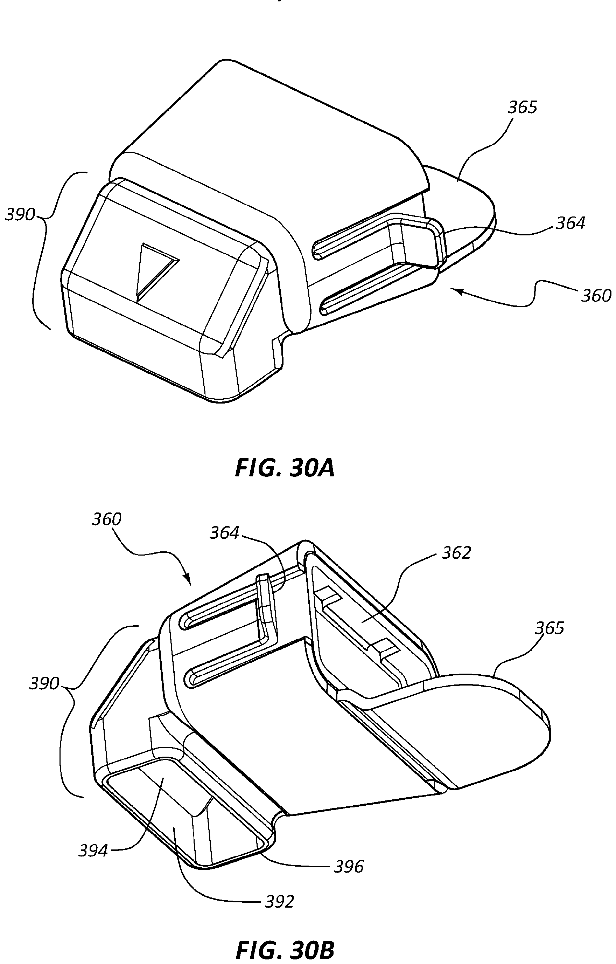

FIGS. 30A and 30B depict a probe cap 360 according to another example, wherein the cap body defines a cavity 362 for receiving therein the head 32 of the ultrasound probe 12 and an engagement feature 364 to secure the cap body to the probe 12. A stabilization arm 365 extends from the cap body so as to enable the cap 360 (and the probe 12 received therein) to be secured to the patient via a band wrapped around the stabilization arm and the arm of the patient, for instance.

As shown, the probe cap 360 further includes a deflector portion 390 for deflecting an ultrasound signal both emanating from and travelling to the transducer of the ultrasound probe 12. The deflector portion 390 is formed as part of the probe cap 360 and defines a channel 392 and an aperture 396 through which ultrasound signals can pass. The deflector portion 390 further includes a deflecting surface 394 disposed in the channel 392. In the present embodiment the deflecting surface 394 is disposed at an angle of about 45 degrees with respect to the transducer surface of the probe head 32 so as to deflect ultrasound signals emanating therefrom through an angle of about 90 degrees, though the deflecting surface can be positioned in other embodiments at other angles so as to produce different resulting angles of signal deflection with respect to the probe transducer.

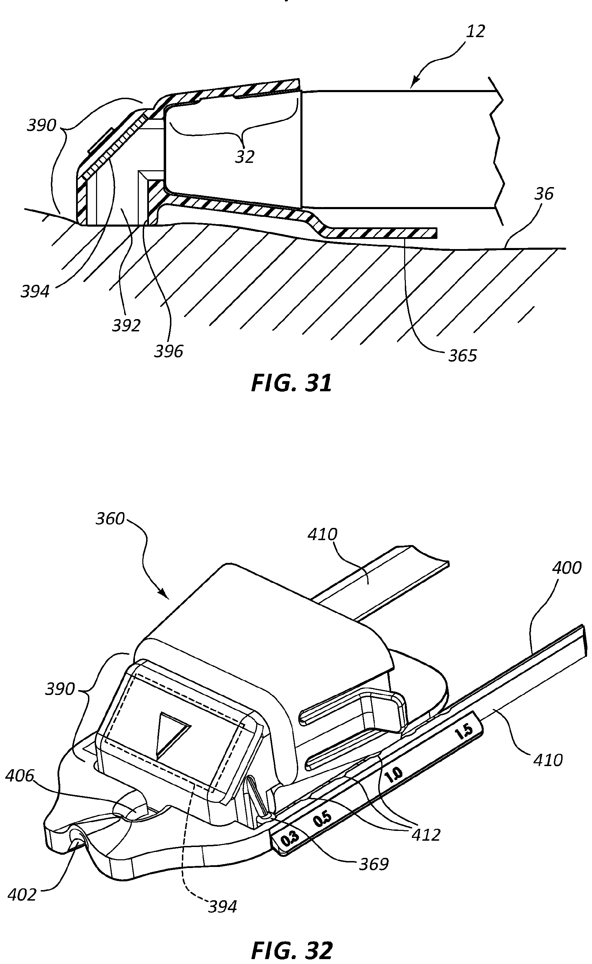

FIG. 31 shows the probe cap 360 positioned against the skin 356 of a patient such that signals emanating from the transducer of the probe head 32 travel through the channel 392, are deflected by the deflecting surface 394, and are directed downward into the body of the patient. Ultrasound signals reflected by an imaged target within the body and received into the channel 392 are also similarly deflected by the deflecting surface 394 toward the probe head 32 for receipt by the transducer. The deflecting surface 394 can include any suitable material having a suitable density so as to reflect the ultrasonic signals travelling through the channel 392. In one example, the deflecting surface includes a plastic material. Also, in one example, the channel 392 is at least partially filled with an ultrasonically transmissive medium, such as an ultrasound gel. In another embodiment, a hydrogel-based spacer component can be disposed in the channel 392, as in previous embodiments. In yet another example, the deflector portion can be integrated into the probe head itself, without the presence of a probe cap. Use of the deflecting probe cap 360 enables the probe 12 to be positioned parallel to the skin 36 of the patient, thus eliminating the need for the clinician to hold the probe upright during use.

FIG. 32 shows that the deflecting probe cap 360 in one example can be included as part of an assembly similar to that shown in FIGS. 29A-29D, wherein the cap body is selectively movable between two rails 410 of a bracket 400. The rails 410 each include corresponding slots 412 for receipt of tabs 369 included on the cap body so as to position the probe cap at one of a plurality of possible distances from a needle guide 402 included on the bracket 400. As before, an observation hole 406 is included proximate the needle guide 402. As described further above in connection with FIGS. 29A-29D, the assembly shown in FIG. 32 enables vessels at a variety of subcutaneous depths to be ultrasonically imaged and accessed by a needle disposed in the fixed-angle needle guide 402 by moving the bracket 400 with respect to the probe cap 360 such that the needle intercepts the imaged vessel at the intended depth.

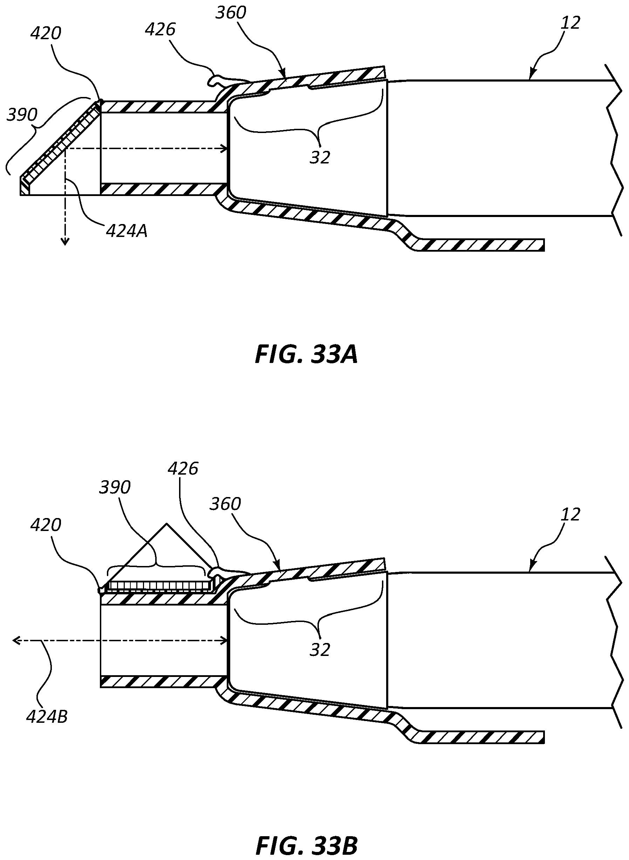

FIGS. 33A and 33B depict the deflecting probe cap 360 according to one example, wherein the deflector portion 390 is hingedly connected to the remainder portion of the cap body via a hinge component 420, including a mechanical or living hinge for instance. So configured, the deflector portion can be selectively positioned so as to deflect ultrasound signals along a deflected signal path 424A (FIG. 33A), or rotated out of the ultrasound signal path (FIG. 33B) so as to enable the ultrasound signals to travel along an undeflected signal path 424B. A latch 426 or other suitable modality can be included to selectively secure the deflector portion 390 in place. Note that in one embodiment a deflecting probe cap can be adjustable such that deflection of the ultrasound signal can be achieved through a variety of angles.

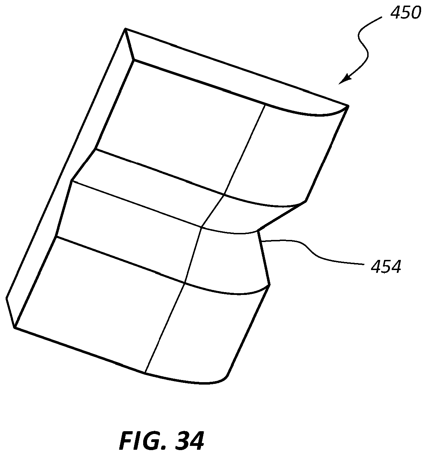

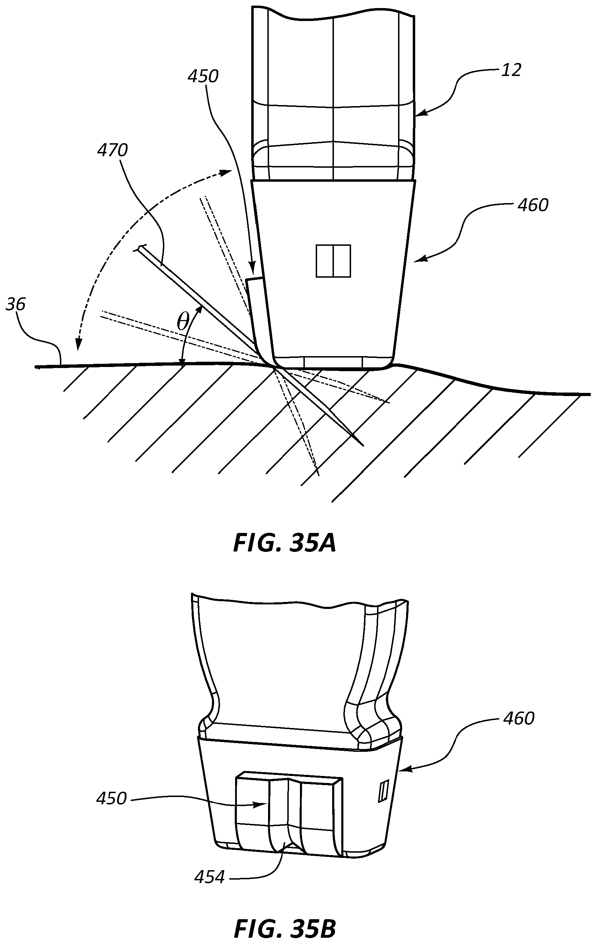

FIG. 34 shows a needle guide 450 according to one example that can be employed with one or more of the probe caps described herein, such as the probe cap 460 shown in FIG. 35B, or can be attached directly to the ultrasound probe. As shown, the needle guide 450 includes a curved, V-shaped open channel 454 that centers a needle therein yet enables the clinician to continuously adjust the angle of attack 0 for the needle at the insertion site during needle insertion, as shown in FIG. 35A. Note that the shape of the channel can vary from what is shown and described.

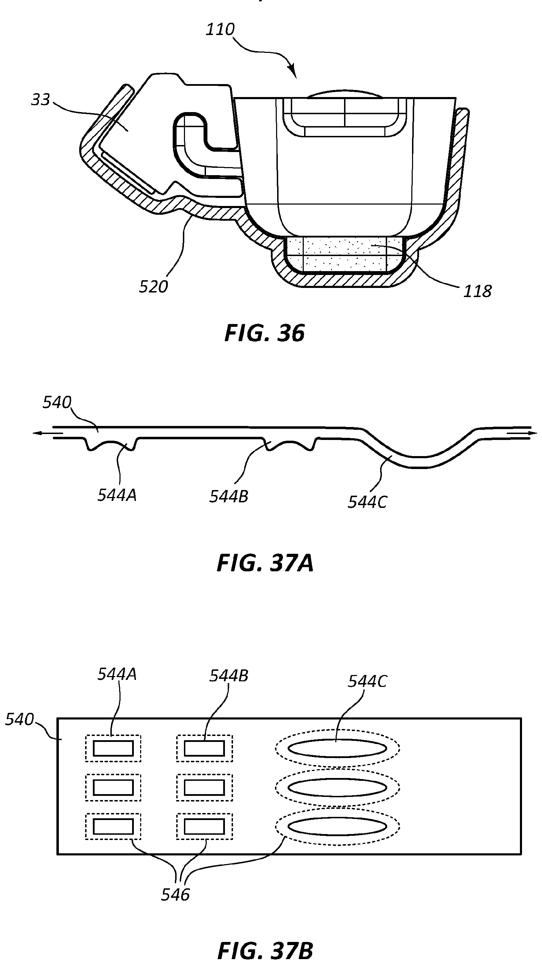

FIGS. 36-47 depict details regarding coupling structures for ultrasonically coupling the head of an ultrasound probe with the patient's skin according to various embodiments. Such ultrasonic coupling (also referred to herein as acoustic coupling) enables the ultrasound signals produced by the head of the ultrasound probe to pass through the coupling structure and the patient's skin in order to penetrate, impinge on, and reflect from the patient's subcutaneous tissue. This in turn enables suitable ultrasound images of the subcutaneous tissue to be taken. In the examples to be described, the coupling structures can in many instances be adapted so as to fit handheld and other probes of varying sizes and configurations.

FIG. 36 according to one embodiment in accordance with the present invention shows a cap 110 configured similarly to other caps described above and configured for removable attachment to a head portion of an ultrasound probe, such as the head portion 32 of the ultrasound probe 12 (FIGS. 24A, 24B). The cap 110 includes the above-described spacer component 118, also referred to herein as an insert (such as a hydrogel insert) or a coupling component, attached therewith and extending from a bottom surface thereof. The insert spacer component 118 can include any one of a variety of acoustically transparent materials including naturally-based and synthetic hydrogels. The spacer component 118 is positioned such that it is interposed between the head of the ultrasound probe and the patient's skin to enable ultrasound signals to pass therethrough. A needle guide 33, also described further above, is attached to the probe cap 110 to assist in guiding a needle into the patient to intercept an ultrasonically imaged vessel or other subcutaneous feature.

In accordance with the present embodiment, a second coupling component that also serves to ultrasonically couple the probe head with the patient's skin is also disclosed. As shown in FIG. 36, the second coupling component includes a sock 520 that is disposed about the probe cap 110, spacer component 118, and needle guide 33. In the present embodiment, the sock 520 includes a natural or synthetic hydrogel or other acoustically transparent material that is suitably compliant so as to fit over the aforementioned components, though in other embodiments a lesser portion of the cap and/or needle guide may be covered by the sock. In greater detail, a portion of the sock 520 covers the hydrogel spacer component 118; being acoustically transparent, the sock nonetheless enables ultrasound signals to pass therethrough. Thus, ultrasound signals emitted and received by the head of the ultrasound probe can readily pass through both the spacer component 118 and the sock 520. So configured, the spacer component 118 of the present embodiment serves as a first coupling component, while the sock 520 serves as a second coupling component.

The sock 520 is configured to enable the probe 12 (*e.g.,* FIGS. 24A, 24B) to be employed in performing an ultrasonic pre-scan with the sock in place as shown in FIG. 36. Once the pre-scan has been completed, the area of the patient's skin where the pre-scan took place can be washed and cleaned prior to needle insertion. The sock 520 can then be removed from the cap 110 and the remaining spacer component 118 - which is sterile due to it being previously covered by the sock - can be placed against the cleaned area of the patient's skin to perform a subsequent ultrasound scan immediately prior to inserting the needle into the skin.

Note that the sock can be sized so to fit one or more of differing probe and/or cap sizes. In one embodiment, the sock is defined from a sheet of commercially available fiber-reinforced or unreinforced hydrogel material that can be shaped before being cured via light or other radiation, temperature, etc. to define the desired sock shape. In one embodiment, the sock is defined from an initially flat sheet that is forced into the desired shaped via a heating process. Other suitable materials can be employed to define the sock, including polyethylene oxide, silicone, thermoplastic elastomers ("TPE"), etc. In another embodiment, not forming part of the present invention, the inner spacer component is removed and only the hydrogel sock is included with the cap assembly. In one embodiment, characteristics of the sock material include acoustic transparency with respect to the spacer component, lubricity, and durability. Glycerin, silicone oil, and other lubricants can also be employed in this and other embodiments. In another embodiment, the sock can be pre-attached to the probe cap and used as a mold for pouring and molding the hydrogel spacer component. Note that in one embodiment, the spacer component and/or the sock can include a hydrogel component such as is described in U.S. Patent Application No. 13/671,382, filed November 7, 2012, and entitled "Ruggedized Ultrasound Hydrogel Insert".

FIGS. 37A and 37B depict various details of coupling components according to another example, wherein a mold is employed into which a natural or synthetic hydrogel or other suitable solution can be poured then cured to form a sheet 540 that defines a plurality of hydrogel (in the present embodiment) coupling components, or spacer components 544, similar to the spacer component 118 shown in FIG. 36. In detail, FIGS. 37A and 37B show spacer components of various sizes/configurations, namely, spacer components 544A, 544B, and 544C, so as to fit ultrasound probes of varying sizes. In the present example, the spacer components 544A, B, and C include perforations 546 defined thereabout so as to ease their removal from the hydrogel sheet 540 after formation.

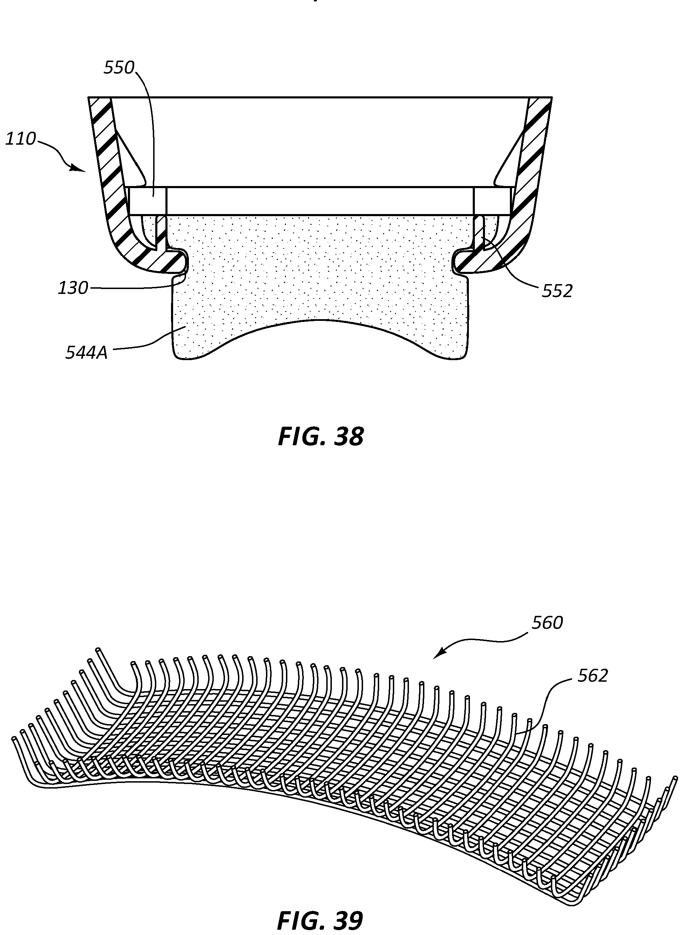

Once formed, the spacer components 544A, B, and C can be removed from the sheet 540 using the perforations 546, then inserted into caps, such as the probe cap 110 shown in FIG. 38, or other coupling structure. In detail, FIG. 38 according to one embodiment shows one of the spacer components 544A disposed in the aperture 130 of the probe cap 110 and retained in place by a retention ring 550. Retention posts 552 and/or other retention features can also be used to secure the spacer component 544A in place. Note that the shape and configuration of the spacer component can be specific to a particular probe head/cap, or generic so as to fit a range of probes. Indeed, in one example the hydrogel spacer component discussed here can be coupled to a sheath configured to cover the ultrasound probe. The sheath can include a clasp or other suitable fixation device at/near a proximal end thereof so as to clamp on to the probe or cable of the probe and keep the spacer component in place on the head of the probe.

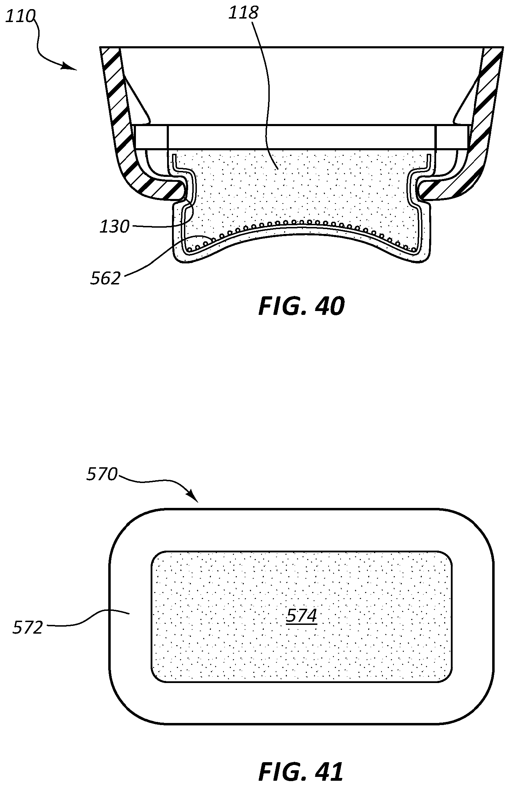

FIG. 39 shows a substantially rigid frame, or reinforcement structure 560 that is employed in a coupling component according to another example. As shown, the reinforcement structure 560 includes a mesh-like lattice component 562 configured to generally conform to the shape of a hydrogel or other suitable insert, such as the spacer component 118 shown in FIG. 40. In the present example and as shown in FIG. 40, the lattice component 562 is incorporated into the spacer component 118 and is shaped so as to help secure the spacer component 118 to the probe cap 110 so as to prevent displacement or removal of the spacer component from the probe cap.

In one example, the lattice component 562 includes a suitable material, such as polyethylene, polyamides such as nylon, polyethylene oxide, etc. In one embodiment, the material for the lattice component 562 is chosen so as to be acoustically transparent and/or matching the acoustic transparency of the hydrogel insert, which in one embodiment, includes a density substantially near that of water. In another embodiment, though not fully acoustically transparent, the mesh size of the lattice component is small enough diameter so as to present a suitable acoustic transparency. As used herein, "acoustically transparent" includes the ability for ultrasound signals to freely pass through the subject medium without significant attenuation. The lattice component 562 is sufficiently rigid so as to prevent shredding or breakup of the hydrogel coupling component, in one embodiment, thus enabling the coupling component to be moved across the skin without significant structural compromise.

FIG. 41 shows a coupling structure according to another example, wherein the coupling structure is implemented as an ultrasound imaging patch 570 including an adhesive portion 572 disposed about the patch perimeter and an imaging region 574 disposed in the central region. The patch 570 is configured to be adhered to the skin at the imaging site by the adhesive portion 572. The imaging region 574 includes a fibrous or other suitable base material impregnated with gel or other acoustically transparent material that enables an ultrasound probe to image the patient's body therethrough when the head portion thereof is placed in contact with the imaging region. In one example , the base material includes a woven natural or synthetic fiber and the gel impregnated therein includes a natural or synthetic hydrogel. Once the body portion has been imaged through the imaging region 574, a needle or other device can penetrate through the imaging patch 570 into the patient's body without first removing the patch, in one embodiment. Also, in one example, the patch 70 can include medication, pain relief or anesthetics, etc. to aid in patient care/comfort. In yet another embodiment, a hole can be defined in the patch into which a hydrogel insert can be disposed.

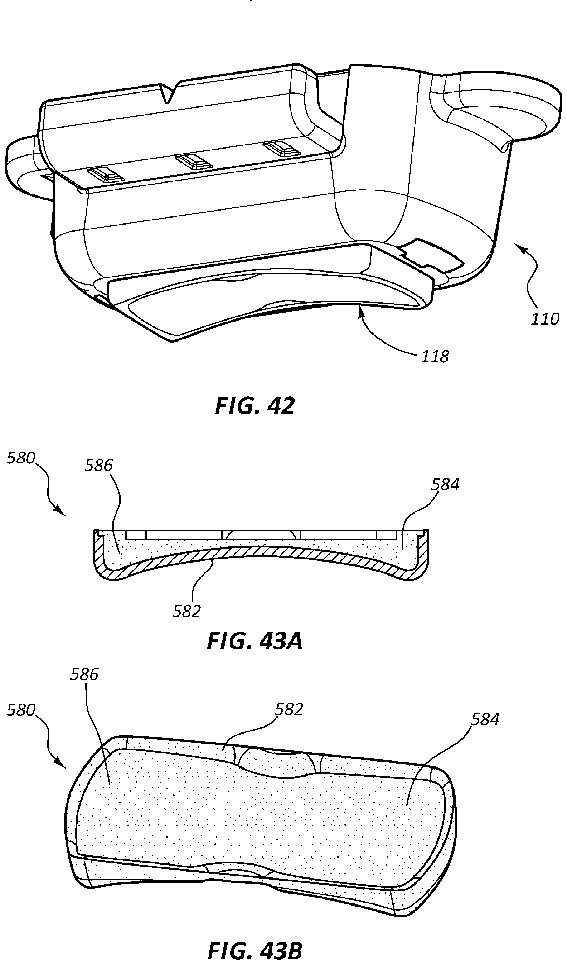

Yet another coupling structure is depicted in FIG. 42, which shows the probe cap 110 including the spacer component 118 through which ultrasound signals are sent and received by the ultrasound probe when the cap is attached thereto. The spacer component 118 in the present embodiment includes an acoustically transparent, elastomeric, nonadhesive hot-melt material that can serve to enable sliding of the probe cap 82 over the skin of the patient without the use of ultrasound gel being first applied to the skin. In one embodiment, suitable lubricants and/or softeners can be added to the hot-melt material of the spacer component 118 during manufacture thereof so as to improve its compliant nature and ease its movement over the skin surface during later use. EVA copolymers, polyolefins, polyamides, polyesters, polyurethanes are non-limiting examples of materials the spacer component 118 may include, in one embodiment.

In another embodiment, the spacer component 118 of the cap 110 can include an injection-moldable, acoustically transparent material and can be molded separately before joined to the cap or molded directly to the cap in a two-shot injection molding process. The material of the spacer component 118 can include a thermoplastic elastomer ("TPE"), silicone, etc. In one embodiment, the lubricity of this or other spacer components described herein can be improved by including a suitable coating, such as a hydrophilic or parylene coating, for instance. The coating can be included on one or both of the skin-contacting portion of the spacer component and the portion that contacts the head portion of the ultrasound probe. This in turn can assist in ultrasonically coupling the spacer component 118 to the probe head portion and in enabling smooth movement of the spacer component over the skin surface. In one embodiment, the spacer component 118 and probe cap 110 can be packaged in a sterile solution, such as sterile saline. When the cap 110 is later removed from the sterile solution for use, the spacer component 118 maintains its lubrication via the residual sterile saline solution disposed thereon. In another embodiment, the saline solution can be added to surface of the spacer component 118 by the user immediately prior to use.

FIGS. 43A and 43B depict details of a coupling component according to another example, wherein a spacer component 580 for attachment to a probe cap (such as the probe cap 110 of FIG. 42) includes a compliant outer membrane 582 that is shaped for attachment to the probe cap about its aperture. The membrane 582 is further configured to retain therein an acoustically transparent substance, such as a hydrogel in a cavity 584 defined by the membrane. In the present example, the cavity 584 of the outer membrane 582 is filled with hydrogel before or after membrane attachment to the probe cap. Later during ultrasound probe use, the probe head portion is ultrasonically coupled to the patient's skin surface via the hydrogel-filled membrane 582 of the spacer component 580. Isolation of the hydrogel from the patient's skin in this manner prevents degradation, flaking, or other degradation of the hydrogel during use against the patient's skin. The membrane 582 can include any suitable, compliant material, including TPEs or silicone, for instance.

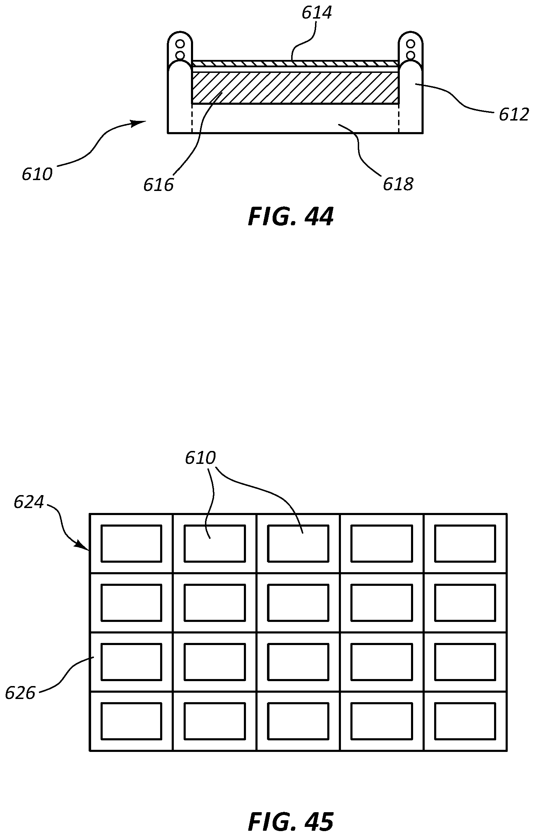

FIG. 44 depicts details of a coupling structure according to another example. FIG. 44 shows a probe cap 610 including a body 612 that is configured to be removably attached to a head portion of an ultrasound probe, though in another embodiment the probe cap - as with the other caps disclosed herein - can be configured for permanent attachment to the probe head. The cap body 612 includes a flexible barrier 614 that is deformed by the head portion when the cap 610 is attached to the probe. A reservoir 616 is defined by the cap body 612 and is filled with a solution that can include hydrogel, bactericide, lubricant, etc. An osmotic membrane 618 is disposed at the bottom of the cap 610 and is in fluid communication with the reservoir 616.

During use, the cap 610 is attached to the head portion of the ultrasound probe such that the barrier 614 is deformed. Deformation of the barrier 614 causes pressure to be exerted on the fluid in the reservoir 616. This in turn causes the fluid to penetrate through the osmotic membrane 618 and on to the patient's skin, where it can be used to lubricate cap movement over the skin, disinfect the skin, etc. The barrier 614, reservoir fluid, and osmotic membrane 618 in the present embodiment are configured to be substantially acoustically transparent so as to permit passage of ultrasound signals therethrough.

FIG. 45 shows a plurality of probe caps 610 arranged atop a sheet 626 of osmotic membrane in an array 624 in one manufacturing configuration, according to one example. Of course, varying manufacturing processes could also be employed.

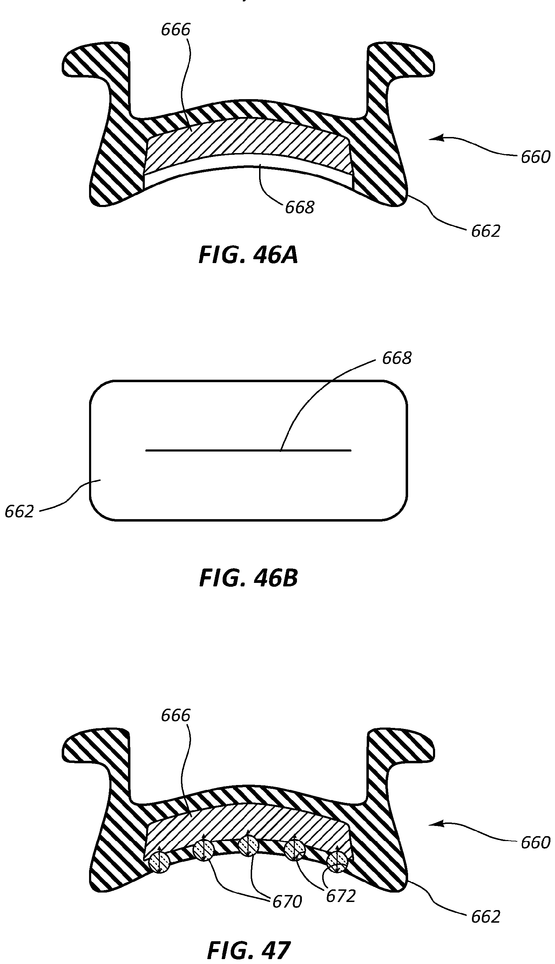

FIGS. 46A and 46B depict various details of a coupling structure according to another example. In detail, FIGS. 46A and 46B show a probe cap 660 including a body 662 that is configured to be removably attached to a head portion of an ultrasound probe. The cap body 612 includes a flexible barrier that is interposed between the probe head portion and a reservoir 666 of the body. The flexible barrier is deformed by the head portion when the cap 660 is attached to the probe. The reservoir 666 is filled with a solution that can include hydrogel, bactericide, lubricant, etc. A slit valve 668 is disposed at the bottom of the cap 660 and is in fluid communication with the reservoir 666.

During use, the cap 660 is attached to the head portion of the ultrasound probe such that the barrier is deformed. Deformation of the barrier causes pressure to be exerted on the fluid in the reservoir 666. Attachment of the cap 660 to the head portion also deforms the slit valve 668, which in turn enables fluid to penetrate therethrough and on to the patient's skin, where it can be used to lubricate cap movement over the skin, disinfect the skin, etc. The barrier, reservoir fluid, and slit valve 668 in the present embodiment are configured to be substantially acoustically transparent so as to permit passage of ultrasound signals therethrough.

The cap 660 in the embodiment shown in FIG. 47 replaces the slit valve of FIGS. 46A and 46B with a plurality of spheres 670 that are each movably disposed in holes 672, with each hole being in fluid communication with the reservoir fluid. The spheres 670 can be made from any suitable material, including plastic, metal, etc. When the cap 660 is pressed against the patient's skin, the spheres 670 are temporarily displaced upward, which enables the reservoir fluid to escape around the spheres through the holes 672 and on to the patient's skin. When skin pressure is removed, the spheres 670 return to seat within the holes 672 and prevent further fluid escape. In addition to the slit valve and spheres discussed in connection with FIGS. 46A-47, other fluid escape mechanisms and configurations can be included, such as perforations, a plurality of holes, etc., as appreciated by one skilled in the art.

The described embodiments are to be considered in all respects only as illustrative, not restrictive. The scope of the present invention is, therefore, defined by the appended claims rather than by the foregoing description.

## Claims

1. A cover for an ultrasound probe, comprising:
a cap (110) that removably attaches to a head portion (32) of the ultrasound probe (12);
a first coupling component (118) included with the cap (110) and capable of ultrasonically coupling the head portion (32) to a skin surface of a patient; and
a second coupling component (520) that removably covers the first coupling component (118) and is capable of ultrasonically coupling the head portion (32) to the skin surface, the first coupling component (118) being adapted for being placed against a patient's skin to perform an ultrasound scan with the second coupling component (520) removed.

2. The cover as defined in claim 1, wherein the second coupling component (520) covers the first coupling component (118) so as to prevent contamination of the first coupling (118) component before removal of the second coupling component (520) and/or
wherein at least one of the first and second coupling components (118, 520) includes a hydrogel.

3. The cover as defined in one of the preceding claims, wherein the second coupling component (520) includes a compliant sheet and envelops at least a portion of the cap (110) together with the first coupling component (118),
wherein the cap (110) preferably further includes a needle guide (33), and wherein the second coupling component (520) covers at least a portion of the needle guide (33).

4. The cover as defined in one of the preceding claims, wherein the second coupling component (520) is pre-installed substantially over the entirety of the cap (110) and is sterilized and packaged before use with the ultrasound probe (12).

5. The cover of one of the preceding claims, wherein the first coupling component (118) includes at least one of naturally-based and synthetic hydrogels.

## Patentansprüche

1. Abdeckung für eine Ultraschallsonde, umfassend:
eine Kappe (110), die abnehmbar an einem Kopfabschnitt (32) der Ultraschallsonde (12) angebracht wird;
eine erste Kopplungskomponente (118), die mit der Kappe (110) eingeschlossen ist und imstande ist, den Kopfabschnitt (32) per Ultraschall an einer Hautoberfläche eines Patienten zu koppeln; und
eine zweite Kopplungskomponente (520), die die erste Kopplungskomponente (118) abnehmbar abdeckt und imstande ist, den Kopfabschnitt (32) per Ultraschall an der Hautoberfläche zu koppeln, wobei die erste Kopplungskomponente (118) angepasst ist, um an der Haut eines Patienten platziert zu werden, um eine Ultraschalluntersuchung mit der zweiten Kopplungskomponente (520) abgenommen durchzuführen.

2. Abdeckung nach Anspruch 1, wobei die zweite Kopplungskomponente (520) die erste Kopplungskomponente (118) derart abdeckt, dass Kontaminierung der ersten Kopplungskomponente (118) vor Abnahme der zweiten Kopplungskomponente (520) verhindert wird, und/oder
wobei mindestens eine der ersten und zweiten Kopplungskomponente (118, 520) ein Hydrogel einschließt.

3. Abdeckung nach einem der vorstehenden Ansprüche, wobei die zweite Kopplungskomponente (520) einen nachgiebigen Bogen einschließt und mindestens einen Abschnitt der Kappe (110) gemeinsam mit der ersten Kopplungskomponente (118) umgibt,
wobei die Kappe (110) vorzugsweise weiter eine Nadelführung (33) einschließt und wobei die zweite Kopplungskomponente (520) mindestens einen Abschnitt der Nadelführung (33) abdeckt.

4. Abdeckung nach einem der vorstehenden Ansprüche, wobei die zweite Kopplungskomponente (520) im Wesentlichen über der Gesamtheit der Kappe (110) vorinstalliert ist und vor Verwendung mit der Ultraschallsonde (12) sterilisiert und verpackt wird.

5. Abdeckung nach einem der vorstehenden Ansprüche, wobei die erste Kopplungskomponente (118) mindestens eines von einem Hydrogel auf natürlicher Basis und einem künstlichen Hydrogel einschließt.

## Revendications

1. Couvercle pour une sonde ultrasonore, comprenant :
une coiffe (110) qui se fixe de manière amovible sur une partie de tête (32) de la sonde ultrasonore (12) ;
un premier composant de couplage (118) inclut avec la coiffe (110) et capable de coupler par ultrasons la partie de tête (32) à une surface de peau d'un patient ; et
un second composant de couplage (520) qui recouvre de manière amovible le premier composant de couplage (118) et est capable de coupler par ultrasons la partie de tête (32) à la surface de la peau, le premier composant de couplage (118) étant adapté pour être placé contre la peau d'un patient pour effectuer un balayage ultrasonore avec le second composant de couplage (520) retiré.

2. Couvercle selon la revendication 1, dans lequel le second composant de couplage (520) recouvre le premier composant de couplage (118) de manière à empêcher une contamination du premier composant de couplage (118) avant le retrait du second composant de couplage (520) et/ou
dans lequel au moins un des premier et second composants de couplage (118, 520) inclut un hydrogel.

3. Couvercle selon l'une des revendications précédentes, dans lequel le second composant de couplage (520) inclut une feuille souple et enveloppe au moins une partie de la coiffe (110) avec le premier composant de couplage (118),
dans lequel la coiffe (110) inclut en outre de préférence un guide d'aiguille (33), et dans lequel le second composant de couplage (520) recouvre au moins une partie du guide d'aiguille (33).

4. Couvercle selon l'une des revendications précédentes, dans lequel le second composant de couplage (520) est pré-installé sensiblement sur la totalité de la coiffe (110) et est stérilisé et conditionné avant utilisation avec la sonde ultrasonore (12).

5. Couvercle selon l'une des revendications précédentes, dans lequel le premier composant de couplage (118) inclut au moins un parmi des hydrogels naturels et synthétiques.
